# EUROPEAN PATENT SPECIFICATION

(11) **EP 2 804 669 B1**
(45) Date of publication and mention of the grant of the patent: **29.03.2023**
(21) Application number: 13816242.5
(22) Date of filing: 18.01.2013
(51) Int. Cl.: A61K 31/22, A61K 31/23, A61P 1/08, A61P 25/28, A61P 21/00

(54) **COMPOSITIONS AND METHODS OF USE OF PHORBOL ESTERS FOR THE TREATMENT OF STROKE**
ZUSAMMENSETZUNGEN UND VERFAHREN ZUR VERWENDUNG VON PHORBOLESTERN ZUR BEHANDLUNG VON SCHLAGANFALL
COMPOSITIONS ET PROCÉDÉS D'UTILISATION D'ESTERS DE PHORBOL POUR LE TRAITEMENT DE L'AVC

(30) Priority: 18.01.2012 US 201261588162 P; 18.01.2012 US 201261588165 P; 18.01.2012 US 201261588167 P
(43) Date of publication of application: 26.11.2014
(73) Proprietor: Biosuccess Biotech Co. Ltd., San Jose CA 95131 (US)
(72) Inventor: HAN, Zheng, Tao, Eugene, OR 97401 (US); CHEN, Hung-Fong, Taipei 10471 (TW)
(74) Representative: Marks & Clerk LLP
(86) International application number: PCT/US2013/022325
(87) International publication number: WO 2014/011209

(56) References cited:
- WO-A1-2008/094657
- WO-A1-2010/015029
- WO-A2-2004/028516
- WO-A2-2010/059245
- WO-A2-2013/110006
- US-A- 6 063 814
- US-A1- 2007 009 529
- US-A1- 2011 243 917
- US-A1- 2011 245 307
- US-B1- 6 184 248
- DALE SCHAAR ET AL: "A phase I clinical trial of 12- O-tetradecanoylphorbol-13-acetate for patients with relapsed/refractory malignancies", CANCER CHEMOTHERAPY AND PHARMACOLOGY, SPRINGER, BERLIN, DE, vol. 57, no. 6, 1 June 2006 (2006-06-01), pages 789-795, XP019334359, ISSN: 1432-0843, DOI: 10.1007/S00280-005-0125-1
- Protein kinase C-alpha activation by phorbol ester induces secretion of gelatinase B/MMP-9 through ERK 1/2 pathway in capillary endothelial cells- https://pubmed.ncbi.nlm.nih.gov/12469196/ - Abstract
- "How epilepsy can affect memory", , April 2019 (2019-04), Retrieved from the Internet: URL:https://epilepsysociety.org.uk/living- epilepsy/wellbeing/memory/how-epilepsy-can -affect-memory#:~:text=Abnormalities%20in% 20the%20temporal%20or,and%20remembering%20 facts%20for%20exams.
- ALBERTO MAUD: "Memory loss after stroke", NEUROLOGY, vol. 67, 23 October 2006 (2006-10-23), pages E14-E15, DOI: https://doi.org/10.1212/01.wnl.0000244752. 95386.de

## Description

### Technical Field

The present invention relates generally to phorbol esters for use in the treatment and prevention of the symptoms of stroke and the effects of stroke.

### Background

Plants have historically served many medicinal purposes. The World Health Organization (WHO) estimates that 4 billion people, 80% of the world's population, presently use herbal medicine for some aspect of primary health care. (WHO Fact sheet Fact sheet N°134, December 2008) However, it can be difficult to isolate the specific compound in a plant that has the desired medicinal effect and to reproduce it on a commercial scale. Additionally, while the active compound may be isolated from a plant, the other parts of a plant such as the minerals, vitamins, volatile oils, glycosides, alkaloids, bioflavanoids, and other substances may also be involved in the functioning of the active compound or the medicinal effect for which the plant is known, making the use, purification and commercialization of plant based pharmaceutical agents a challenge.

Phorbol is a natural, plant-derived organic compound of the tigliane family of diterpenes. It was first isolated in 1934 as a hydrolysis product of croton oil derived from the seeds of *Croton tiglium,* a leafy shrub of the Euphorbiaceae family that is native to Southeastern Asia. Various esters of phorbol have important biological properties including the reported ability to mimic diacylglycerols and activate protein kinase C (PKC); and to modulate downstream cell signaling pathways including the mitogen-activated protein kinase (MAPK) pathways. Phorbol esters are additionally thought to bind to chimaerins, the Ras activator RasGRP, and the vesicle-priming protein Munc-13 (Brose N, Rosenmund C., JCell Sci;115:4399-411 (2002)). Some phorbol esters also induce nuclear factor-kappa B (NF-κB). The most notable physiological property of phorbol esters is their reported capacity to act as tumor promoters. (Blumberg, 1988; Goel, G et al., Int, Journal of Toxicology 26, 279-288 (2007)).

12-O-tetradecanoylphorbol-13-acetate (TPA), also called phorbol-12-myristate-13-acetate (PMA), is a phorbol ester used in models of carcinogenesis as an inducer for differentiation and/or apoptosis in multiple cell lines and primary cells. TPA has also been reported to cause an increase in circulating white blood cells and neutrophils in patients whose bone marrow function has been depressed by chemotherapy (Han Z. T. et al. Proc. Natl. Acad. Sci. 95, 5363-5365 (1998)), and to inhibit an HIV- induced cytopathic effect on MT-4 cells. (Mekkawy S. et al., Phytochemistry 53, 47-464 (2000)). However, due to a variety of factors, including caustic reactions when contacted with the skin and concerns for its potential toxicity, TPA has not been shown to be an effective tool for treating, managing, or preventing disease. Indeed, as phorbol esters play a key role in activation of protein kinase C, which triggers various cellular responses resulting in inflammatory responses and tumor development (Goel et al., Int, Journal of Toxicology 26, 279-288 (2007)), phorbol esters would generally be excluded from possible treatment candidates for conditions that involve inflammatory reactions such as stroke.

Heart disease and stroke kill some 17 million people a year, causing almost one-third of all deaths globally. They are predicted to become the leading cause of both death and disability worldwide, with the number of fatalities projected to increase to over 20 million a year by 2020 and to 24 million a year by 2030. (Atlas of Heart Disease and Stroke, World Health Organization 2004).

While there are over 300 risk factors associated with coronary heart disease and stroke (Atlas of Heart Disease and Stroke, World Health Organization 2004), in developed countries, at least 1/3 of all cardiovascular disease is attributable to tobacco use, alcohol use, high blood pressure, high cholesterol and obesity.

Current treatments for the management and prevention of stroke are generally a combination of medications such as ACE inhibitors, aspirin, beta blockers and lipid lowering medications; devices such as pacemakers, implantable defibrillators, coronary stents, prosthetic valves and artificial hearts; and operations such as coronary artery bypass, balloon angioplasty, valve repair and replacement, heart transplants and artificial heart operations. "Increasingly, high-technology procedures are chosen over less expensive, but nevertheless effective , strategies " (Atlas of Heart Disease and Stroke, World Health Organization 2004) adding to the rising costs of health care and leading to marked disparities in the quality of treatment between different groups of individuals.

However, even where advanced technology and facilities are available, 60% of those who suffer a stroke die or become dependent and each stroke significantly increases the risk of further episodes. "Worldwide, treatment of cardiovascular diseases and their risk factors remains inadequate for most patients." (Atlas of Heart Disease and Stroke, World Health Organization 2004). There is therefore clearly a need for new and more effective measures to treat and prevent strokes and to treat or prevent the long term effects caused by stroke.

WO2004/028516 (Haller et al) describes the use of agents impeding the expression and/or activity of protein kinase C-alpha (PKC-α) for the treatment of patients with diabetes and complications such as diabetic nephropathy, retinopathy or neuropathy. US2007/009529 (Karpatkin et al) describes a number of agents that induce platelet fragmentation which include phorbol-12-myristate 13-acetate (PMA).

### Summary

Aspects of the present invention are set out in the appended claims. In particular there is provided a phorbol ester of Formula (I) or a pharmaceutically acceptable salt or enantiomer thereof for use in a method for preventing or treating one or more of the effects of stroke in a mammalian subject, or a composition comprising a phorbol ester of Formula I, or a pharmaceutically-acceptable salt or enantiomer thereof for use in a method for the prevention or treatment of one or more effects of stroke in a mammalian subject according to claim 1.

There is further provided a phorbol ester of Formula I, or a pharmaceutically-acceptable salt or enantiomer thereof for use in a method for preventing or treating one or more symptoms of a stroke in a mammalian subject, or a composition comprising a phorbol ester of Formula I, or a pharmaceutically-acceptable salt or enantiomer thereof for use in a method for preventing or treating one or more symptoms of a stroke in a mammalian subject according to claim 10.

Effects of stroke that may be prevented or treated by using the phorbol esters and derivatives of phorbol esters as described herein include paralysis, spatial impairment, impaired judgment, left-sided neglect, memory loss, aphasia, coordination and balance problems, nausea, vomiting, cognitive impairment, perception impairment, orientation impairment, homonymous hemianopsia and impulsivity.

Successful treatment will be determined according to conventional methods, such as a reduction in the severity or sequelae of a stroke, and /or a decrease or elimination of the effects of stroke.

The phorbol esters as described herein may be used to modulate cell signaling pathways. Such modulation may have a variety of results, for example, the use of compositions containing phorbol esters may alter the release of Th1 cytokines in mammalian subjects. In a further example, compositions containing phorbol esters may alter the release of interleukin 2 (IL-2) in mammalian subjects. In an additional example, compositions containing phorbol esters may alter the release of interferon in mammalian subjects. In yet another example, compositions containing phorbol esters may alter the rate of ERK phosphorylation.

The phorbol ester of Formula I is shown below: 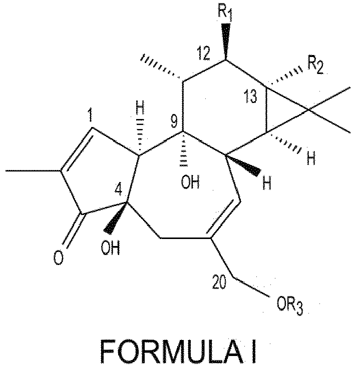 wherein R₁ and R₂ are independently hydrogen; hydroxyl; 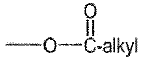 , wherein the alkyl group contains 1 to 15 carbon atoms; 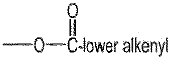 , wherein a lower alkenyl group contains between 1 to 7 carbon atoms; 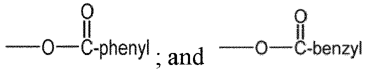 . R₃ is independently hydrogen or 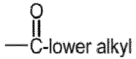 wherein the lower alkyl group contains 1 to 7 carbon atoms . The compositions for use of the present invention further include any pharmaceutical salts and enantiomers, of the compositions of Formula I.

In some embodiments, at least one of R₁ and R₂ are other than hydrogen and R₃ is hydrogen or 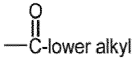 wherein the lower alkyl group contains 1 to 7 carbon atoms . In another embodiment, either R₁ or R₂ is 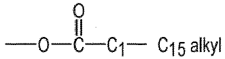 the remaining R₁ or R₂ is a 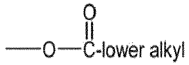 , wherein a lower alkyl is between 1 and 7 carbons, and R₃ is hydrogen.

The alkyl, alkenyl, phenyl and benzyl groups of the formulas of Formula I herein may be unsubstituted or substituted with halogens, preferably, chlorine, fluorine or bromine; nitro; amino; and/or similar type radicals.

An exemplary phorbol ester composition as used in the methods and compositions as described herein is 12-O-tetradecanoylphorbol-13-acetate (TPA) of Formula II, below:

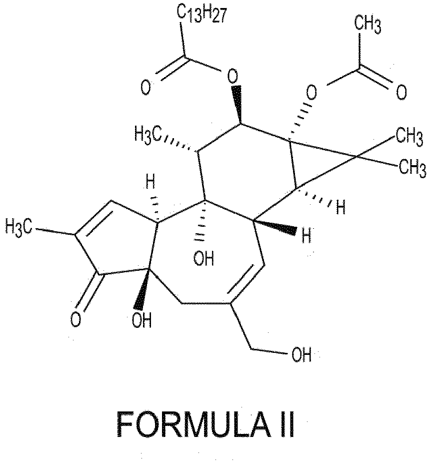

Useful phorbol esters of Formula I within the formulations for uses according to the invention include other pharmaceutically acceptable active salts of said compounds, as well as enantiomers, polymorphs, solvates and/or hydrates of said compounds. Exemplary forms of phorbol esters for use according to the invention include, but are not limited to, phorbol 13-butyrate; phorbol 12-decanoate; phorbol 13-decanoate; phorbol 12,13-diacetate; phorbol 13,20-diacetate; phorbol 12,13-dibenzoate; phorbol 12,13-dibutyrate; phorbol 12,13-didecanoate; phorbol 12,13-dihexanoate; phorbol 12,13-dipropionate; phorbol 12-myristate; phorbol 13-myristate; phorbol 12-myristate-13-acetate (also known as TPA or PMA); phorbol 12,13,20-triacetate; 12-deoxyphorbol 13-angelate; 12-deoxyphorbol 13-angelate 20-acetate; 12-deoxyphorbol 13-isobutyrate; 12-deoxyphorbol 13-isobutyrate-20-acetate; 12-deoxyphorbol 13-phenylacetate; 12-deoxyphorbol 13-phenylacetate 20-acetate; 12-deoxyphorbol 13-tetradecanoate; phorbol 12-tigliate 13-decanoate; 12-deoxyphorbol 13-acetate; phorbol 12-acetate; and phorbol 13-acetate.

Mammalian subjects amenable to treatment with phorbol esters of Formula I, particularly TPA, include, but are not limited to, individuals who have suffered or are at risk for a stroke. Subjects amenable to treatment with phorbol esters of Formula I, particularly TPA, additionally include those suffering from the effects of a stroke including, but not limited to, paralysis, spatial impairment, impaired judgment, left-sided neglect, memory loss, aphasia, coordination and balance problems, nausea, vomiting, cognitive impairment, perception impairment, orientation impairment, homonymous hemianopsia and impulsivity.

These and other subjects are effectively treated prophylactically and/or therapeutically, by administering to the subject an effective amount of a phorbol ester of Formula I sufficient to prevent or treat paralysis, increase spatial awareness, decrease memory loss, decrease aphasia, increase coordination and balance, and improve cognition.

Therapeutically useful formulations for uses of the invention will effectively use a phorbol ester of Formula I in a variety of forms, as noted above, including any active, pharmaceutically acceptable salts of said compounds, as well as enantiomers, polymorphs, solvates, hydrates, and/or combinations thereof. TPA of formula II is employed as an illustrative embodiment of the invention within the examples herein below.

Combinatorial formulations for uses according to the invention are provided which employ an effective amount of a phorbol ester of Formula I in combination with one or more secondary or adjunctive active agent(s) that is/are combinatorially formulated or coordinately administered with the phorbol ester compound of Formula I to yield an effective response in the subject.

Combinatorial formulations for uses according to the invention employ a phorbol ester compound of Formula I in combination with one or more additional, stroke preventing, treating or other indicated, secondary or adjunctive therapeutic agents. The secondary or adjunctive therapeutic agents used in combination with a phorbol ester, e.g., TPA, in these embodiments may possess direct or indirect effects on prevention or recovery from stroke, alone or in combination with the phorbol ester, e.g. TPA; may exhibit other useful adjunctive therapeutic activity in combination with, e.g. TPA (such as anti-clotting, anticholesterolemic, vasodilating, antihypertensive, arteriolar resistance lowering, venous capacity increasing, heart oxygen demand reducing, heart rate decreasing, heart rate stabilizing, or neuroprotecting); or may exhibit adjunctive therapeutic activity useful for treating or preventing stroke or associated symptoms alone or in combination with, e.g. TPA. Such secondary or adjunctive therapeutic agents may be administered prior to, simultaneously, or after administration of a phorbol ester of Formula I.

Useful adjunctive or secondary therapeutic agents in these combinatorial formulations include, but are not limited to, tissue plasminogen activators, anticoagulants, statins, angiotensin II receptor blockers, angiotensin-converting enzyme inhibitors, anti-platelet agents, beta-blockers, aspirin, fibrates, calcium channel blockers, or diuretics. In addition, adjunctive or secondary therapies may be used such as, but not limited to, surgical intervention including carotid endarterectomy, angioplasty, balloon angioplasty, valve repair and replacement, coronary artery bypass, stent placement, craniotomy, endovascular coil embolization, or patent foramen ovale closure.

The foregoing and additional objects, features, aspects and advantages of the present invention will become apparent from the following detailed description.

### Detailed Description

Compositions have been identified for use in preventing and/or treating one or more of the effects of stroke and one or more of the symptomsof stroke in mammalian subjects, including humans.

The compositions for uses as described herein may increase the release of Th1 cytokines, increase ERK phosphorylation, modulate NF-κB activity, prevent or treat paralysis, increase spatial awareness, decrease memory loss, decrease aphasia, increase coordination and balance, improve cognition, improve orientation, and decrease impulsivity.

Formulations for uses according to the invention employ a phorbol ester of Formula I as more fully described in U.S. Patent Application No. 12/023,753, filed January 31, 2008, which claims priority benefit of United States Provisional patent application Serial No. 60/898,810, filed January 31, 2007, 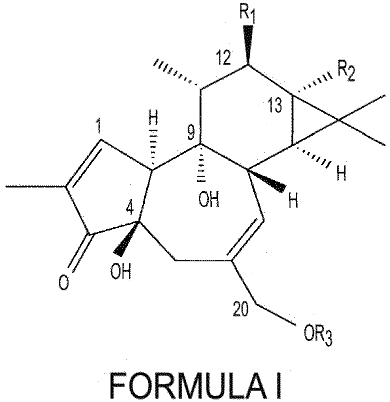 wherein R₁ and R₂ are independently hydrogen; hydroxyl; 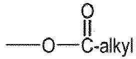 , wherein the alkyl group contains 1 to 15 carbon atoms; 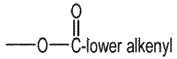 , wherein a lower alkenyl group contains between 1 to 7 carbon atoms; 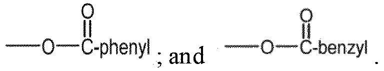 R₃ is hydrogen or 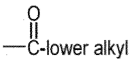 wherein the lower alkyl group contains 1 to 7 carbon atoms .

In some embodiments, at least one of R₁ and R₂ are other than hydrogen and R₃ is hydrogen or 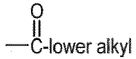 wherein the lower alkyl group contains 1 to 7 carbon atoms . In another embodiment, either R₁ or R₂ is 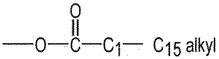 the remaining R₁ or R₂ is a 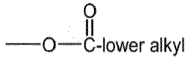 , wherein a lower alkyl is between 1 and 7 carbons, and R₃ is hydrogen.

The alkyl, alkenyl, phenyl and benzyl groups of the formulas herein may be unsubstituted or substituted with halogens, preferably, chlorine, fluorine or bromine; nitro; amino; and/or similar type radicals.

Stroke treating formulations provided herein employ a phorbol ester of Formula I, above, including all active pharmaceutically acceptable compounds of this description as well as various foreseen and readily provided salts , solvates, enantiomers, polymorphs and combinations thereof as anti-stroke agents.

Within the compositions for uses of the invention, one or more phorbol ester compound(s) of Formula I as disclosed herein is/are effectively formulated or administered as an agent effective for treating and preventing one or more of the effects of stroke or one or more of the symptoms of stroke. In exemplary embodiments, TPA is demonstrated for illustrative purposes to be an effective agent in pharmaceutical formulations for use in the described therapeutic methods, alone or in combination with one or more adjunctive therapeutic agent(s). The present disclosure further provides additional, pharmaceutically acceptable phorbol ester compounds in the form of a native or synthetic compound, salts, solvates, enantiomers, and polymorphs, of the compounds disclosed herein, and combinations thereof, which are effective as therapeutic agents in the treatment and prevention of one or more of the effects of stroke or one or more of the symptoms of stroke.

Strokes are caused by disruption of the blood supply to the brain. This may result from either blockage (ischaemic stroke) or rupture of a blood vessel (haemorrhagic stroke). The symptoms of a stroke include sudden numbness or weakness, especially on one side of the body; sudden confusion or trouble speaking or understanding speech; sudden trouble with walking, dizziness, or loss of balance or coordination. Risk factors for stroke include high blood pressure, abnormal blood lipids, tobacco use, physical inactivity, obesity, stress, diabetes, alcohol use, excess homocystein in the blood, inflammation and abnormal coagulation. There are also non-modifiable risk factors such as age, heredity, gender and ethnicity.

Treatment for stroke has three distinct phases: prevention, therapy immediately after a stroke, and post-stroke rehabilitation. The compositions for uses described herein may be used at any phase of stroke treatment, independently or in conjunction with one or more additional therapies including other pharmaceutical agents, devices or surgical interventions.

Phorbol is a natural, plant-derived polycyclic alcohol of the tigliane family of diterpenes. It was first isolated in 1934 as the hydrolysis product of croton oil derived from the seeds of *Croton tiglium.* It is well soluble in most polar organic solvents and in water. Esters of phorbol have the general structure of Formula I, below: 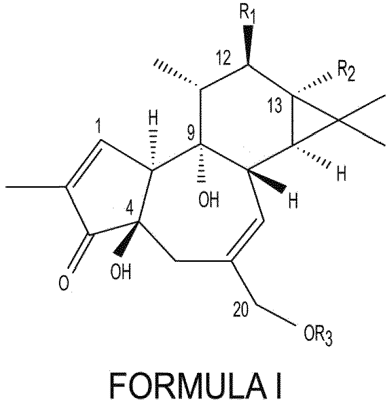 wherein R₁ and R₂ are selected from the group consisting of hydrogen; hydroxyl; 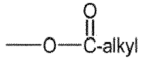 , wherein the alkyl group contains 1 to 15 carbon atoms; 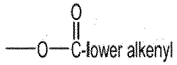 , wherein a lower alkenyl group contains between 1 to 7 carbon atoms; 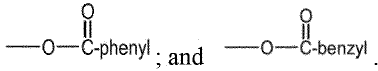 R₃ is hydrogen or 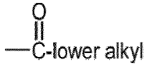 wherein the lower alkyl group contains 1 to 7 carbon atoms as well as pharmaceutically acceptable salts, enantiomers, polymorphs, solvates and hydrates of compounds of Formula I.

The term "lower alkyl" or "lower alkenyl" as used herein means moieties containing 1 to 7 carbon atoms. In the compounds of the Formula I, the alkyl or alkenyl groups may be straight or branched chain. In some embodiments, either or both R₁ or R₂, are a long chain carbon moiety (i.e., Formula I is decanoate or myristate).

The alkyl, alkenyl, phenyl and benzyl groups of the formulas herein may be unsubstituted or substituted with halogens, preferably, chlorine, fluorine or bromine; nitro; amino and similar type radicals.

Organic and synthetic forms of phorbol esters, including any preparations or extracts from herbal sources such as *croton tiglium,* are contemplated as useful compositions comprising phorbol esters (or phorbol ester analogs, related compounds and/or derivatives) for use within the embodiments herein.

In more detailed embodiments, illustrative structural modifications according to Formula I above will be selected to provide useful candidate compounds for treating and/or preventing damage due to stroke and/ or managing the effects or sequelae of strokes in mammalian subjects, including humans, wherein: at least one of R₁ and R₂ are other than hydrogen and R₃ is selected from the group consisting of hydrogen or 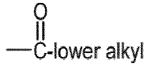 wherein the lower alkyl group contains 1 to 7 carbon atoms . In another embodiment, either R₁ or R₂ is 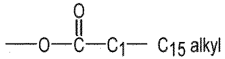 the remaining R₁ or R₂ is 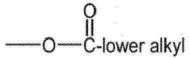 and R₃ is hydrogen.

An exemplary embodiment of a phorbol ester compound of Formula I useful in the treatment of in treating or preventing damage from strokes, and/or managing the effects or sequelae of strokes in mammalian subjects, including humans, is found in phorbol 12-myristate-13-acetate (also known as PMA or 12-O-tetradecanoyl-phorbol-13-acetate (TPA)) shown in Formula II, below.

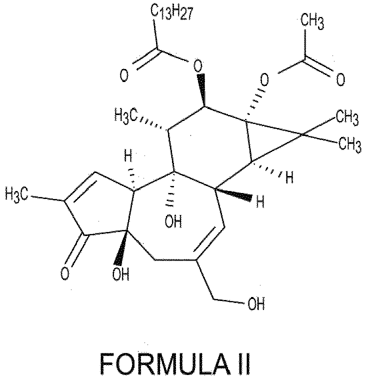

Additional useful phorbol esters within the formulations for uses of the invention include, but are not limited to, other pharmaceutically acceptable active salts of said compounds, as well as active enantiomers, polymorphs, solvates, and/or hydrates of said compounds. Further exemplary forms of phorbol esters for use according to the invention include, but are not limited to, phorbol 13-butyrate; phorbol 12-decanoate; phorbol 13-decanoate; phorbol 12,13-diacetate; phorbol 13,20-diacetate; phorbol 12,13-dibenzoate; phorbol 12,13-dibutyrate; phorbol 12,13-didecanoate; phorbol 12,13-dihexanoate; phorbol 12,13-dipropionate; phorbol 12-myristate; phorbol 13-myristate; phorbol 12,13,20-triacetate; 12-deoxyphorbol 13-angelate; 12-deoxyphorbol 13-angelate 20-acetate; 12-deoxyphorbol 13-isobutyrate; 12-deoxyphorbol 13-isobutyrate-20-acetate; 12-deoxyphorbol 13-phenylacetate; 12-deoxyphorbol 13-phenylacetate 20-acetate; 12-deoxyphorbol 13-tetradecanoate; phorbol 12-tigliate 13-decanoate; 12-deoxyphorbol 13-acetate; phorbol 12-acetate; and phorbol 13-acetate as shown in Table 1.

**Table 1**

| Exemplary Phorbol Esters | |
|---|---|
| Phorbol 13-Butyrate | 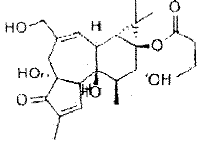 |
| Phorbol 12-Decanoate | 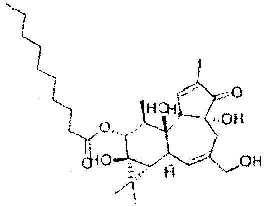 |
| Phorbol 13-Decanoate | 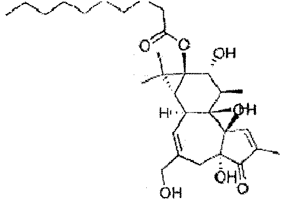 |
| Phorbol 12,13-Diacetate | 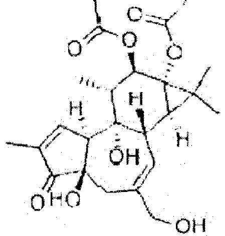 |
| Phorbol 13,20-Diacetate | 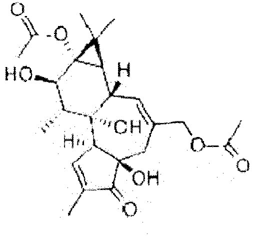 |
| Phorbol 12,13-Dibenzoate | 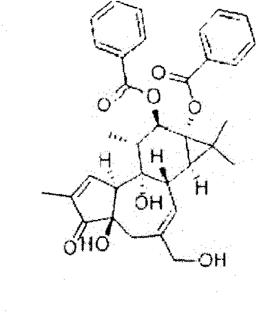 |
| Phorbol 12,13-Dibutyrate | 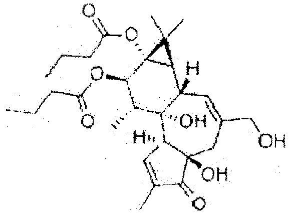 |
| Phorbol 12,13-Didecanoate | 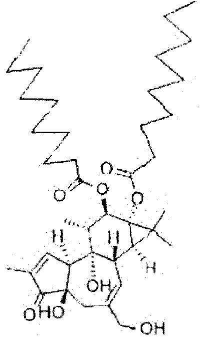 |
| Phorbol 12,13-Dihexanoate | 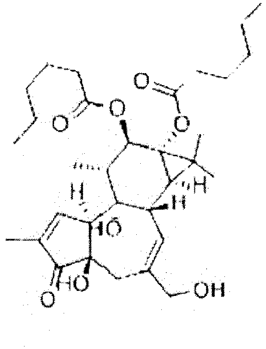 |
| Phorbol 12,13-Dipropionate | 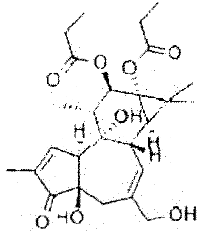 |
| Phorbol 12-Myristate | 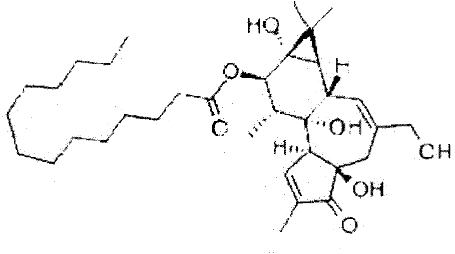 |
| Phorbol 13-Myristate | 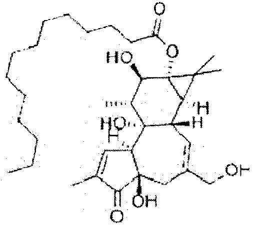 |
| Phorbol 12-Myristate-13-Acetate (also known as TPA or PMA) | 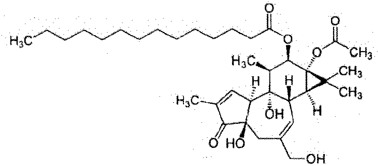 |
| Phorbol 12,13,20-Triacetate | 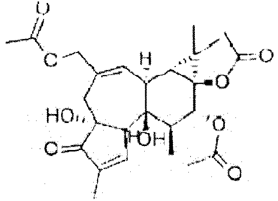 |
| 12-Deoxyphorbol 13-Angelate | 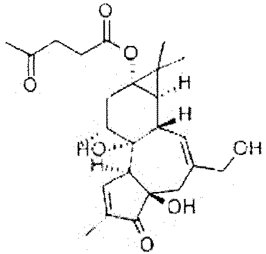 |
| 12-Deoxyphorbol 13-Angelate 20-Acetate | 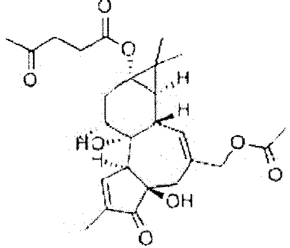 |
| 12-Deoxyphorbol 13-Isobutyrate | 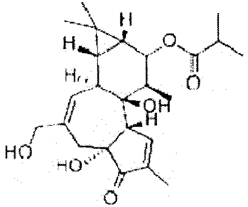 |
| 12-Deoxyphorbol 13-Isobutyrate-20-Acetate | 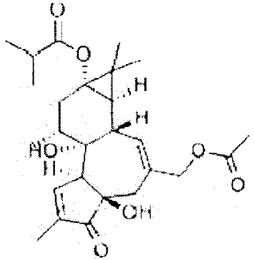 |
| 12-Deoxyphorbol 13-Phenylacetate | 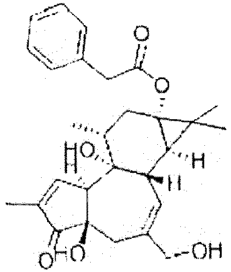 |
| 12-Deoxyphorbol 13-Phenylacetate 20-Acetate | 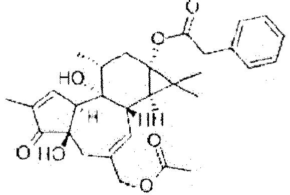 |
| 12-Deoxyphorbol 13-Tetradecanoate | 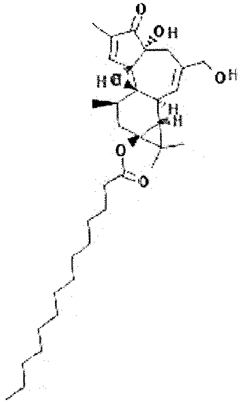 |
| Phorbol 12-Tigliate 13-Decanoate | 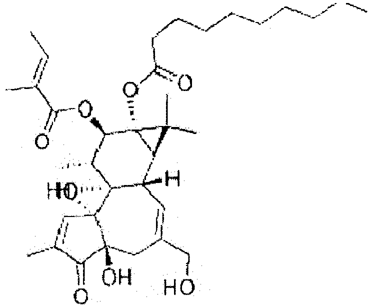 |
| 12-Deoxyphorbol 13-Acetate | 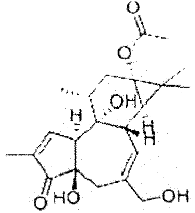 |
| | |
| Phorbol 12-Acetate | 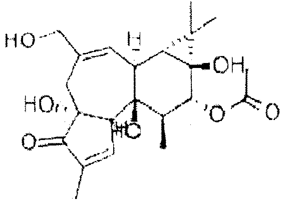 |
| Phorbol 13-Acetate | 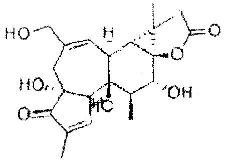 |

Compositions for uses as described herein comprise stroke treating compositions comprising a stroke damage alleviating or preventing effective amount of a phorbol ester compound of Formula I, which is effective for prophylaxis and/or treatment stroke related symptoms or sequelae in a mammalian subject. A "stroke treating," "anti-clotting," "anticholesterolemic," "vasodilating," "antihypertensive," "arteriolar resistance lowering," " venous capacity increasing," "heart oxygen demand reducing," "heart rate decreasing," " heart rate stabilizing," or "neuroprotective" effective amount of the active compound is therapeutically effective, in a single or multiple unit dosage form, over a specified period of therapeutic intervention, to measurably alleviate one or more symptoms or sequelae of stroke in the subject. The compositions for uses of the invention are effective in treatment methods to prevent or alleviate symptoms of stroke or sequelae of stroke in human and other mammalian subjects vulnerable to or who have suffered a stroke.

Phorbol ester treating compositions for uses of the invention comprise an effective amount or unit dosage of a phorbol ester compound of Formula I, which may be formulated with one or more pharmaceutically acceptable carriers, excipients, vehicles, emulsifiers, stabilizers, preservatives, buffers, and/or other additives that may enhance stability, delivery, absorption, half-life, efficacy, pharmacokinetics, and/or pharmacodynamics, reduce adverse side effects, or provide other advantages for pharmaceutical use. The unit dosage amount of the phorbol ester of Formula (I) for uses according to the invention is from about 10 to about 1500 µg. Other suitable effective unit dosage amounts of the active compounds for administration to mammalian subjects, including humans, may range from about 20 to about 1000 µg, about 25 to about 750 µg, about 50 to about 500 µg, about 150 to about 500 µg, about 125 µg to about 500 µg, about 180 to about 500 µg, about 190 to about 500 µg, about 220 to about 500 µg, about 240 to about 500 µg, about 260 to about 500 µg, about 290 to about 500 µg. In certain embodiments, the disease treating effective dosage of a phorbol ester compound or related or derivative compound of Formula I may be selected within narrower ranges of, for example, 10 to 25 µg, 30-50 µg, 75 to 100 µg, 100 to 300 µg, or 150 to 500 µg. These and other effective unit dosage amounts may be administered in a single dose, or in the form of multiple daily, weekly or monthly doses, for example in a dosing regimen comprising from 1 to 5, or 2 to 3, doses administered per day, per week, or per month. In one exemplary embodiment, dosages of 10 to 30 µg, 30 to 50 µg, 50 to 100 µg, 100 to 300 µg, or 300 to 500 µg, are administered one, two, three, four, or five times per day. In more detailed embodiments, dosages of 50-100 µg, 100-300 µg, 300-400 µg, or 400-600 µg are administered once or twice daily. In a further embodiment, dosages of 50-100 µg, 100-300 µg, 300-400 µg, or 400-600 µg are administered every other day. In alternate embodiments, dosages are calculated based on body weight, and may be administered, for example, in amounts from about 0.5/µg/m² to about 300µg/m² per day, about 1 µg/m² to about 200 µg/m², about 1 µg/m² to about 187.5 µg/m² per day, about 1 µg/m² per day to about 175 µg/m² per day, about 1 µg/m² per day to about 157 µg/m² per day about 1 µg/m² to about 125 µg/m² per day, about 1 µg/m² to about 75 µg/m² per day, 1 µg/m² to about 50/ µg/m² per day, 2 µg/m² to about 50 µg/m² per day, 2 µg/m² to about 30 µg/m² per day or 3 µg/m² to about 30 µg/m² per day.

In other embodiments, dosages may be administered less frequently, for example, 0.5µg/m² to about 300µg/m² every other day, about 1 µg/m² to about 200 µg/m², about 1 µg/m² to about 187.5 µg/m² every other day, about 1 µg/m² to about 175 µg/m² every other day, about 1 µg/m² per day to about 157 µg/m² every other day about 1 µg/m² to about 125 µg/m² every other day, about 1µg/m² to about 75 µg/m² every other day, 1 µg/m² to about 50µg/m² every other day, 2 µg/m² to about 50 µg/m² every other day, 2 µg/m² to about 30 µg/m² per day or 3 µg/m² to about 30 µg/m² per day. In additional embodiments, dosages may be administered 3 times/week, 4 times/week, 5 times/week, only on weekdays, only in concert with other treatment regimens, on consecutive days, or in any appropriate dosage regimen depending on clinical and patient-specific factors.

The amount, timing and mode of delivery of compositions for uses of the invention comprising an (alternatively "stroke treating," "anti-clotting," "anticholesterolemic," "vasodilating," "antihypertensive," "arteriolar resistance lowering," " venous capacity increasing," "heart oxygen demand reducing," "heart rate decreasing," " heart rate stabilizing," "ERK phorsphorylation inducing," "IL-2 modulating" and/or "neuroprotective") effective amount of a phorbol ester compound of Formula I, will be routinely adjusted on an individual basis, depending on such factors as weight, age, gender, and condition of the individual, the acuteness of the disease and/or related symptoms, whether the administration is prophylactic or therapeutic, and on the basis of other factors known to effect drug delivery, absorption, pharmacokinetics, including half-life, and efficacy.

An effective dose or multi-dose treatment regimen for the instant disease treating (alternatively, "stroke treating," "anti-clotting," "anticholesterolemic," "vasodilating," "antihypertensive," "ERK phorsphorylation inducing," "arteriolar resistance lowering," " venous capacity increasing," "heart oxygen demand reducing," "heart rate decreasing," " heart rate stabilizing," "blood clot decreasing," "neuroprotective," "IL-2 modulating" or "NFκB modulating") formulations for uses of the invention will ordinarily be selected to approximate a minimal dosing regimen that is necessary and sufficient to substantially prevent or alleviate the symptoms of stroke in the subject. A dosage and administration protocol will often include repeated dosing therapy over a course of several days or even one or more weeks or years. An effective treatment regime may also involve prophylactic dosage administered on a day or multi-dose per day basis lasting over the course of days, weeks, months or even years.

Effectiveness of the compositions for uses of the invention in the treatment of stroke may be demonstrated using a variety of model systems including temporary middle cerebral artery occlusion as shown in Example 9, permanent middle cerebral artery occlusion as shown in Example 8, endovascular filament middle cerebral artery occlusion, embolic middle cerebral artery occlusion as shown in Example 7, endothelin-1 -induced constriction of arteries and veins, or cerebrocortical photothrombosis. The phorbol ester compositions for uses of the present invention will decrease the symptoms or long term effects exhibited by the model systems by 0%, 20%, 30%, 50% or more, up to a 75-90%, 96% or greater decrease over control animals.

Effectiveness of the compositions for uses of the invention in the treatment of stroke may further be demonstrated by a decrease in the symptoms exhibited in individuals who have suffered a stroke. Such symptoms includeparalysis, spatial impairment, impaired judgment, left-sided neglect, memory loss, aphasia, coordination and balance problems, nausea, vomiting, cognitive impairment, perception impairment, orientation impairment, homonymous hemianopsia and impulsivity. The phorbol ester compositions for uses of the present invention will decrease the symptoms exhibited by individuals by 0%, 20%, 30%, 50% or more, up to a 75-90%, 96% or greater decrease over initial states.

Combinatorial disease treating ("stroke treating," "anti-clotting," "anticholesterolemic," "vasodilating," "antihypertensive," "ERK phorsphorylation inducing," "arteriolar resistance lowering," " venous capacity increasing," "heart oxygen demand reducing," "heart rate decreasing," " heart rate stabilizing," or "NFκB modulating") formulations for uses according to the invention are provided which employ an effective amount of a phorbol ester compound of Formula I and one or more secondary or adjunctive agent(s) that is/are combinatorially formulated or coordinately administered with the phorbol ester compound of Formula I to yield a combined, multi-active disease treating composition.

Exemplary combinatorial formulations for uses according to the invention employ the phorbol ester compound of Formula I, , in combination with one or more additional, neuroprotective or other indicated, secondary or adjunctive therapeutic agents that is/are useful for treatment or prophylaxis of the targeted disease, condition and/or symptom(s). For most combinatorial formulations , a phorbol ester compound of Formula I is formulated, or coordinately administered, in combination with one or more secondary or adjunctive therapeutic agent(s), to yield a combined formulation that is combinatorially effective or coordinately useful to prevent or treat the effects of stroke. Exemplary combinatorial formulations in this context employ a phorbol ester compound of Formula I, in combination with one or more secondary or adjunctive therapeutic agents selected from tissue plasminogen activator, an anticoagulant, a statin, angiotensin II receptor blockers, angiotensin-converting enzyme inhibitor, anti-platelet agent, fibrate, beta-blocker, calcium channel blocker, or diuretic. Exemplary anticoagulants include, but are not limited to, heparin, warfarin, heparinoids, phenindione, atomentin, acenocoumarol, phenprocoumon, idraparinux, fondaparinux, and thrombin inhibitors. Exemplary statins include, but are not limited to, lovastatin, amlodipine, atorvastatin, rosuvastatin, simvastatin, fluvastatin, pitavastatin, and pravastatin. Exemplary angiotensin II receptor blockers include, but are not limited to, candesartan, eprosartan, irbesartan, losartan, olmesartan, telmisartan, and valsartan. Angiotensin converting enzyme inhibitors include, but are not limited to, enazepril, captopril, enalapril, fosinopril, isinopril, moexipril, perindopril, quinapril, ramipril, and trandolapril. Exemplary beta-blockers include, but are not limited to, alprenolol, bucindolol, carteolol, carvedilol, labetalol, nadolol, oxprenolol, penbutolol, pindolol propranolol, sotalol, timolol, eucommia, acebutolol, atenolol, betaxolol, bisoprolol, celiprolol, esmolol, metoprolol, and nebivolol. Exemplary calcium channel blockers include, but are not limited to, amlodipine, clevidipine, diltiazem, felodipine, isradipine, nifedipine, nicardipine, nimodipine, nisoldipine, and verapamil. Exemplary diuretics include, but are not limited to, chlorothiazide, hydrochlorothiazide, bumetanide, ethacrynic acid, furosemide, amiloride, eplerenone, spironolactone and triamterene. Exemplary fibrates include, but are not limited to, benzafibrate, ciprofibrate, clofibrate, gemfibrozil or fenofibrate. Exemplary anti-platelet agents include, but are not limited to, clopidogrel and ticlopidin.

Compositions for use in coordinate treatment methods may further employ surgical intervention including, but not limited to, the use of pacemakers, implantable defibrillators, coronary stents, prosthetic valves, coronary artery bypass, balloon angioplasty, valve repair and replacement, carotid endarterectomy, angioplasty, stent placement, craniotomy, endovascular coil embolization patent foramen ovale closure and heart transplantation.

In certain embodiments the invention provides combinatorial disease treating ("stroke treating," "anti-clotting," "anticholesterolemic," "vasodilating," "antihypertensive," "ERK phorsphorylation inducing," "arteriolar resistance lowering," " venous capacity increasing," "heart oxygen demand reducing," "heart rate decreasing," " heart rate stabilizing," or "NFκB modulating") formulations comprising a phorbol ester and one or more adjunctive agent(s) having disease treating activity. Within such combinatorial formulations, a phorbol ester of Formula I and the adjunctive agent(s) having disease treating activity will be present in a combined formulation in disease treating ("stroke treating," "anti-clotting," "anticholesterolemic," "vasodilating," "antihypertensive," "ERK phorsphorylation inducing," "arteriolar resistance lowering," " venous capacity increasing," "heart oxygen demand reducing," "heart rate decreasing," " heart rate stabilizing," or "NFκB modulating" ) effective amounts, alone or in combination. In exemplary embodiments, a phorbol ester compound of Formula I and a non-phorbol ester agent(s) will each be present in a disease treating/preventing amount (i.e., in singular dosage which will alone elicit a detectable alleviation of symptoms in the subject). Alternatively, the combinatorial formulation may comprise one or both the phorbol ester compound of Formula I and the non-phorbol ester agents in sub-therapeutic singular dosage amount(s), wherein the combinatorial formulation comprising both agents features a combined dosage of both agents that is collectively effective in eliciting a disease, condition, or symptom alleviating response. Thus, one or both of the phorbol ester of Formula I, , and non-phorbol ester agents may be present in the formulation, or administered in a coordinate administration protocol, at a sub-therapeutic dose, but collectively in the formulation or method they elicit a detectable decrease in symptoms of disease, the occurrence or recurrence of stroke, or sequelae from a stroke in the subject. In yet another embodiment, the combinatorial formulation may include one or more neuroprotective agents. In a further embodiment, the combinatorial formulation may include one or more anti-inflammatory agents or other secondary or additional therapeutic agents as described herein.

To practice coordinate administration methods, a phorbol ester compound of Formula Imay be administered, simultaneously or sequentially, in a coordinate treatment protocol with one or more of the secondary or adjunctive therapeutic agents contemplated herein. Thus, in certain embodiments a compound is administered coordinately with a non-phorbol ester agent, or any other secondary or adjunctive therapeutic agent contemplated herein, using separate formulations or a combinatorial formulation as described above (i.e., comprising both a phorbol ester compound of Formula I or related or derivative compound, and a non-phorbol ester therapeutic agent). This coordinate administration may be done simultaneously or sequentially in either order, and there may be a time period while only one or both (or all) active therapeutic agents individually and/or collectively exert their biological activities.

In another embodiment, compositions for use in such coordinate treatment methods may, for example, follow or be derived from various protocols for the treatment of stroke. Coordinate treatment methods may, for example, include a phorbol ester and/or treatments for prevention or treatment of damage caused by a stroke. A distinguishing aspect of all such coordinate treatment methods is that the phorbol ester compound of Formula I, exerts at least some activity, which yields a favorable clinical response in conjunction with a complementary stroke preventing or treating agent, or distinct, clinical response provided by the secondary or adjunctive therapeutic agent. Often, the coordinate administration of the phorbol ester compound of Formula I with the secondary or adjunctive therapeutic agent will yield improved therapeutic or prophylactic results in the subject beyond a therapeutic effect elicited by the phorbol ester compound of Formula I or the secondary or adjunctive therapeutic agent administered alone. This qualification contemplates both direct effects as well as indirect effects.

Within exemplary embodiments, a phorbol ester compound of Formula I, will be coordinately administered (simultaneously or sequentially, in combined or separate formulation(s)), with one or more secondary stroke treating compounds or other indicated or adjunctive therapeutic agents, e.g. tissue plasminogen activator, an anticoagulant, a statin, angiotensin II receptor blockers, angiotensin-converting enzyme inhibitor, fibrate, beta-blocker, calcium channel blocker, lipid-lowering medication, anti-platelet agent or diuretic. In addition, adjunctive or secondary therapies may be used in the treatment of stroke or the effects of stroke such as, but not limited to, pacemakers, implantable defibrillators, coronary stents, prosthetic valves, artificial hearts, coronary artery bypass, balloon angioplasty, valve repair and replacement, heart transplantation, carotid endarterectomy, angioplasty, stent placement, craniotomy, endovascular coil embolization or patent foramen ovale closure.

As noted above, in all of the various embodiments of the invention contemplated herein, the disease treating formulations may employ a phorbol ester compound of Formula I in any of a variety of forms, including any one or combination of the subject compound's pharmaceutically acceptable salts, solvates, enantiomers, polymorphs, solvates, and/or hydrates. In exemplary embodiments of the invention, TPA is employed within the therapeutic formulations for illustrative purposes.

The pharmaceutical compositions for uses of the present invention may be administered by any means that achieve their intended therapeutic or prophylactic purpose. Suitable routes of administration for the compositions for uses of the invention include, but are not limited to, conventional delivery routes, devices and methods including injectable methods such as, but not limited to, intravenous, intramuscular, intraperitoneal, intraspinal, intrathecal, intracerebroventricular, intraarterial, subcutaneous and intranasal routes.

The compositions for uses of the present invention may further include a pharmaceutically acceptable carrier appropriate for the particular mode of administration being employed. Dosage forms of the compositions for uses of the present invention include excipients recognized in the art of pharmaceutical compounding as being suitable for the preparation of dosage units as discussed above. Such excipients include, without intended limitation, binders, fillers, lubricants, emulsifiers, suspending agents, sweeteners, flavorings, preservatives, buffers, wetting agents, disintegrants, effervescent agents and other conventional excipients and additives.

If desired, the compositions for uses of the invention can be administered in a controlled release form by use of a slow release carrier, such as a hydrophilic, slow release polymer. Exemplary controlled release agents in this context include, but are not limited to, hydroxypropyl methyl cellulose, having a viscosity in the range of about 100 cps to about 100,000 cps or other biocompatible matrices such as cholesterol.

Some phorbol ester compositions of Formula I for uses of the invention are designed for parenteral administration, e.g. to be administered intravenously, intramuscularly, subcutaneously or intraperitoneally, including aqueous and non-aqueous sterile injectable solutions which, like many other contemplated compositions for uses of the invention, may optionally contain anti-oxidants, buffers, bacteriostats and/or solutes which render the formulation isotonic with the blood of the mammalian subject; and aqueous and non-aqueous sterile suspensions which may include suspending agents and/or thickening agents. The formulations may be presented in unit-dose or multi-dose containers. Additional compositions and formulations for uses of the invention may include polymers for extended release following parenteral administration. The parenteral preparations may be solutions, dispersions or emulsions suitable for such administration. The subject agents may also be formulated into polymers for extended release following parenteral administration. Pharmaceutically acceptable formulations and ingredients will typically be sterile or readily sterilizable, biologically inert, and easily administered. Such polymeric materials are well known to those of ordinary skill in the pharmaceutical compounding arts. Parenteral preparations typically contain buffering agents and preservatives, and injectable fluids that are pharmaceutically and physiologically acceptable such as water, physiological saline, balanced salt solutions, aqueous dextrose, glycerol or the like. Extemporaneous injection solutions, emulsions and suspensions may be prepared from sterile powders, granules and tablets of the kind previously described. Preferred unit dosage formulations are those containing a daily dose or unit, daily sub-dose, as described herein above, or an appropriate fraction thereof, of the active ingredient(s).

In more detailed embodiments, compositions for uses of the invention may comprise a phorbol ester compound of Formula I encapsulated for delivery in microcapsules, microparticles, or microspheres, prepared, for example, by coacervation techniques or by interfacial polymerization, for example, hydroxymethylcellulose or gelatin-microcapsules and poly(methylmethacylate) microcapsules, respectively; in colloidal drug delivery systems (for example, liposomes, albumin microspheres, microemulsions, nano-particles and nanocapsules); or within macroemulsions.

As noted above, in certain embodiments the compositions for uses of the invention may employ pharmaceutically acceptable salts, e.g., acid addition or base salts of the above-described phorbol ester compounds of Formula I. Examples of pharmaceutically acceptable addition salts include inorganic and organic acid addition salts. Suitable acid addition salts are formed from acids which form non-toxic salts, for example, hydrochloride, hydrobromide, hydroiodide, sulphate, hydrogen sulphate, nitrate, phosphate, and hydrogen phosphate salts. Additional pharmaceutically acceptable salts include, but are not limited to, metal salts such as sodium salts, potassium salts, cesium salts and the like; alkaline earth metals such as calcium salts, magnesium salts and the like; organic amine salts such as triethylamine salts, pyridine salts, picoline salts, ethanolamine salts, triethanolamine salts, dicyclohexylamine salts, N,N'-dibenzylethylenediamine salts and the like; organic acid salts such as acetate, citrate, lactate, succinate, tartrate, maleate, fumarate, mandelate, acetate, dichloroacetate, trifluoro acetate, oxalate, and formate salts; sulfonates such as methanesulfonate, benzenesulfonate, and p-toluenesulfonate salts; and amino acid salts such as arginate, asparginate, glutamate, tartrate, and gluconate salts. Suitable base salts are formed from bases that form non-toxic salts, for example aluminum, calcium, lithium, magnesium, potassium, sodium, zinc and diethanolamine salts.

### Examples

The experiments described below demonstrate novel and powerful uses for phorbol esters and derivative compounds as stroke treating and preventing agents. These and additional findings are further expanded and elucidated within the following examples. Examples I to V are provided for background understanding and do not fall under the scope of the appended claims.

### Example I

### Effect of TPA on the Peripheral White Blood Cells (WBC) And Hemoglobin (Hb) Counts In S180 Cell-Injected Mice:

Sarcoma 180 (S180) cells were injected into Kwen-Ming mice. On the third day, the mice were given TPA interperitoneally (i.p.). at 50, 100 or 200 µg/kg/day for 7 days. On the second day after the treatment was completed, blood samples were taken from the tails of the treated mice for WBC and Hb analyses. The WBC counts for the treated groups (50, 100, or 200 ug/kg/day for 7 days) were 16.1±7.4, 18.7±.3.0 and 20.7±.3.4 ×10⁹ /L, respectively; the WBC count for the control group was 13.6±1.8×10⁹ /L. The Hb of the treated groups were 136±11, 149±12 and 149±10 g/L, and the Hb of the control group was 134+-15 g/L. The results indicate that i.p. injection of TPA could increase the peripheral WBC counts in mice in a dose-dependent manner, whereas the Hb levels were not greatly affected in TPA treated mice when compared to the control mice.

### Example II

### Dose Ranging Study.

Due to the strong local irritation caused by TPA application, TPA was given to patients by intravenous (i.v.) infusion. TPA solution in a sterile syringe was injected into 200 ml of sterile saline and mixed well for i.v. infusion.

The Toxicity and Side Effects of Different TPA Doses Administered Clinically:

### (1) TPA given at 1 mg/patient/week:

One mg TPA in solution was mixed well with 200 ml of sterile saline for intravenous infusion which was completed in 1 h at the rate of 16 µg/min. One hour after TPA administration, patients started to have chills which lasted for about 30 min, followed by fever, (the patients' temperature reached 37.5-39.5°C. which lasted for 3-5 h, then returned to normal) with light to heavy perspiration. The above symptoms could be alleviated by giving the patients glucocorticoids. TPA at this dose caused a minority of patients to bleed, several patients suffered for a short period of time difficulty in breathing, and Hb was detected in the urine. However, these side effects were short lived and reversible. The cardiac, hepatic, renal and pulmonary functions were all found to be normal.

### (2) TPA given at 0.5 mg/patient × 2/week: (two doses a week)

0.5 mg of TPA in solution was mixed well with 200 ml of saline for intravenous infusion which was completed in 1 h at the rate of 8 µg/min. The reactions after administration were similar to that of the 1 mg TPA dosage, but to a lesser extent than the 1 mg dose. The patients tolerated the lower dose more easily. Occasionally, Hb was detected in patients' urine. Difficulty in breathing was not observed. The cardiac, hepatic, renal and pulmonary functions were all normal.

### (3) TPA given at 0.25 mg/patient × 4/week:

0.25 mg of TPA in solution was mixed well with 200 ml of saline for intravenous infusion which was completed in 1 h at the rate of 4 µg/min. After administration, symptoms such as chills and fever were also observed, but to a much lesser extent than with the higher dosages. No Hb was detected in the urine, and no patient suffered difficulty in breathing. The cardiac, hepatic, renal and pulmonary functions were all normal.

### Example III

### Treatment of Relapsed/Refractory Malignancies with TPA

Patients with histologically documented relapsed/refractory hematologic malignancy/bone marrow disorders are treated with a combination of TPA (Xichuan Pharmaceuticals, Nan Yang, Henan, China), dexamethasone and choline magnesium trisalicylate. Comparable methods as set forth below for demonstrating the therapeutic use of TPA in the treatment of Acute Myelogenous Leukemia (AML) will be applied to demonstrate the use of TPA for treating other neoplastic conditions and malignancies. In addition to the specific protocols herein, successful treatment and/or remission will be determined for different targeted neoplastic and malignant conditions using any of a wide variety of well known cancer detection and assessment methods-for example by determining size reduction of solid tumors, histopathological studies to evaluate tumor growth, stage, metastatic potential, presence/expression levels of histological cancer markers, etc.

AML is an aggressive disease that generally warrants urgent and intensive therapy. The average patient age at AML diagnosis is 64-68 years old, and patients over the age of 60 treated with standard chemotherapy are cured of their disease < 20% of the time. Patients who develop AML after an antecedent hematologic disorder or prior leukemogenic chemotherapy/radiation therapy have similarly poor outcomes, as do patients whose disease is associated with specific adverse cytogenetic and clinical features. Hence, most patients diagnosed with AML have patient and/or disease-related features that are associated with a very poor prognosis. For patients with relapsed disease, no standard non-transplant therapy has demonstrated the capacity for cure. For these patients, AML is often a fatal disease. New approaches to the therapy of AML are needed.

Employing the methods and compositions of the instant invention, TPA, is developed as a therapeutic agent for treating patients with AML, based on TPA's novel role in modulating intracellular signaling pathways, it's capacity to induce differentiation and/or apoptosis in cell lines, and clinical data indicating the effectiveness of TPA in treating neoplastic and malignant disorders, including myeloid malignancies.

Thus far clinical evaluation of TPA has demonstrated that TPA exerts direct therapeutic cytotoxic effects in at least a subset of AML cases, as measured by cell viability and apoptosis assays. In all primary cultures analyzed by Western analysis, TPA strongly induced ERK phosphorylation by 1 hour in culture. TPA's cytotoxic effect on primary AML cells is associated with the subsequent loss of the phospho-ERK pro-survival signal after 24 hour *ex vivo* exposure. This observation is in good agreement with other studies that reported decreased primary AML survival after pharmacological interruption of ERK signaling by MEK inhibitors, such as PD98059, U0126 and PD 184352. In our studies, loss of ERK signaling was associated with induction of ERK phosphatases.

In addition to protein kinase C and ERK activation, TPA is a known inducer of NF-κB, a pro-survival transcription factor often constitutively active in AML blasts and leukemic stem cells. Recent work from our laboratory has demonstrated that AML cell NF-κB can be inhibited *in vivo* with 48h of treatment with dexamethasone + choline magnesium trisalicylate (CMT). In addition, we have shown that dexamethasone can induce MKP-1 ERK phosphatase expression and enhance TPA cytotoxicity on primary AML samples. In this context, we have chosen in exemplary embodiments below to use dexamethasone and CMT as adjunctive medications to be used 24h pre- and 24h post treatment with TPA. These medications are well-tolerated and anticipated to reduce inflammatory adverse effects of treatment and enhance TPA cytotoxicity by increasing ERK phosphatase expression and inhibiting NF-κB. In addition dexamethasone and CMT will be used as adjunctive medications because they are anti-inflammatory, may ameliorate adverse effects, and may enhance anti-leukemic activity by inhibition of the anti-apoptotic effects of constitutive NF-xB expression and induction of phosphatases that decrease signaling pathway activity.

An initial TPA Phase 1 study enrolled 35 patients [23 with relapsed/refractory AML, 2 with other myeloid malignancies (CML-blast crisis, myelodysplasia with excess blasts), 3 with Hodgkin's Disease, 3 with non-Hodgkin's lymphoma and 4 with solid tumors]. The majority of patients had relapsed/refractory AML. Our clinical results include one AML patient with stable disease for > 5 months, who received 8 TPA infusions. In a second AML patient, a pronounced (5-fold) decline in the number of circulating blasts was seen following TPA administration. This decline in leukemic blasts persisted for 4 weeks, and the patient eventually died from a fungal infection. Finally, a patient with relapsed and refractory Hodgkin's disease despite high dose chemotherapy with autologous stem cell rescue had a partial remission of a chest wall mass after TPA administration. TPA dose escalation has been completed, in the last cohort 2 out of 3 patients treated at a dose of 0.188mg/m2 d1-5, 8-12 experienced grade III non-hematologic dose limiting toxicities (DLT), establishing the maximum tolerated TPA dose as a single agent at 0.125mg/m2/d on d1-5 and 8-12.

In the case of AML and other hematologic malignancies, patients are given an initial dose of TPA of 1 mg/week × 3 weeks (days 1, 8, 15) administered with continuous/intermittent pulse oximetry for 6 hours. Twenty four hours prior to initiation of TPA therapy, patients are given 10mg of dexamethasone every six hours and 1500mg of choline magnesium trisalicylate (**CMT**) every eight hours continuing until 24 hours after administration of TPA. After administration of the initial dose of TPA, patients have a two week rest period after which they may be reevaluated. Those patients that have a disease response or stabilization from the initial dose of TPA are treated for up to six cycles of twenty-eight days according to the protocol below.

Following the two week rest period, patients are pre-medicated with Tylenol 650 mg and Benadryl 25-50 mg (depending on the patient's size and age) thirty minutes prior to administration of TPA. They are then given an intravenous infusion of TPA through a central venous catheter daily for 5 days a week for two consecutive weeks followed by a 2-week rest period. TPA is administered at a dose of 1 mg in 200 ml of normal saline over 1 hour. Twenty four hours prior to initiation of TPA therapy, patients are given 10 mg of dexamethasone every six hours and 1500 mg of choline magnesium trisalicylate continuing every eight hours until 24 hours after administration of the TPA.

Blood levels of TPA are measured prior to and after infusion using a bioassay that measures organic solvent extractable differentiation activity. 1ml of blood is extracted twice with 5 ml of ethyl acetate, redissolving the extraction residue in 50 µL of ethanol and addition of an aliquot of HL60 cells. After 48 hours, adherent cells are measured.

Tests are also run on blood samples taken prior to and after infusion with TPA to determine levels of white blood cells, platelets, and neutrophils. The samples are additionally analyzed for the presence of myeloblasts and Auer rods. These and continuing experiments will further elucidate the therapeutic cytotoxic and other effects that TPA elicits against neoplastic cells in AML and other neoplastic and malignant conditions.

### Example IV

### Measurement of the Modulation of ERK Activation

Phospho-ERK levels are measured in circulating malignant cells in patients with leukemia and in peripheral blood mononuclear cells in lymphoma/solid tumor patients. A blood sample is taken from patients treated according to the protocol of Example III both prior to and after administration of TPA.

In leukemia patients with a WBC≥ 1000 per µL, flow cytometry is performed on a blood sample using cell surface antigen-specific and phospho-ERK specific antibodies directly conjugated to flurophores (BD Biosciences, San Jose, CA). Samples are taken pre-administration of TPA and one hour after infusion of TPA on days 1, 2, and 11 in the initial treatment according to the protocol of Example III and days 1 and 11 in subsequent cycles. In leukemia patients with an absolute leukemic blast number ≥2500 per µL and other non-leukemic patients, peripheral blood samples are taken on days 1, 8 and 15 of the initial cycle according to the protocol of Example III prior to and 1 and 4 hours post infusion. Samples are also analyzed using Western blot analysis for phosphor-ERK, and total ERK1/2 levels to confirm the results obtained from the flow cytometry and correlated to clinical responses.

The foregoing analyses will further elucidate TPA's role in treatment of neoplastic and malignant conditions, including TPA's cytotoxic effect on malignant cells, exemplified by primary AML cells, and the associated reduction by TPA of the phosphor-ERK pro-survival signal.

### Example V

### Measurement of NF-κB Modulation

In prior studies we have shown that NF-κB activity can be modulated in patients following administration of TPA with dexamethasone. Additionally, dexamethasone has been shown to induce MKP-1 ERK phosphatase expression and enhance TPA cytotoxicity. The following studies are designed to further elucidate how NF-κB activity is therapeutically modulated in patients treated with TPA plus dexamethasone.

NF-κB binding is measured in patient peripheral blood samples at baseline and pre and post infusion from patients treated with TPA according to Example III using ELISA-based assays (BD Bioscience, San Jose, USA). NF-κB levels are quantified using chemiluminescent intensity to detect binging in limiting amounts of cellular extract using a 96-well format. Additionally, electrophoretic mobility shift assays are performed to measure NF- κB binding in peripheral blood samples from leukemia patient with an absolute leukemic blast number ≥2500 per µL and other non-leukemic patients with normal white blood cell counts.

The foregoing studies will further show that TPA is an inducer of NF-κB; however these experiments demonstrate that AML cell NF-κB can be inhibited with treatment with dexamethasone and choline magnesium trisalicylate.

### Example VI

### Treatment of Individuals Who Have Suffered a Stroke.

Patient N.C., male, 68, suffered a stroke eighteen months prior to treatment with TPA. At the time TPA treatment was initiated, he was unable to walk without a cane, had difficulty with both his left hand and left leg and was tired and weak. He received injections of 1 ampoule containing 0.19mg of TPA (0.125mg/m²) every other day for four weeks, then 0.24 mg of TPA (1.25 × 0.125mg/m²) every other day for 2 weeks, and then 0.26 mg of TPA (1.5 × 0.125mg/m²) every other day for an additional 3 weeks. The patient has recovered fully.

Patient M.C., male, age 65, suffered a stroke seven years prior to beginning treatment with TPA. He received 3-4 injections of 0.19 mg of TPA (0.125mg/m²) per week for ten weeks for a total of 35 injections. He has regained mobility in his face and had an 80% improvement in the mobility of his right side.

### Example VII

### Treatment of Embolic Stroke Model with TPA

Male Sprague-Dawley rats (Charles River, Japan) each having a body weight of 280-350 g are used. An embolic stroke is induced following a modification of the method of Kudo, et al. (1982) The rats to be used for the collection of blood are anesthetized with 1.0% halothane (Fluorothane^{™}; Takeda, Osaka, Japan) under spontaneous respiration. A 24-gauge Surflo^{™} (Terumo Medical Products, Elkton, MD) is secured in the femoral artery and 0.1 mL of arterial blood is taken with a 1-mL syringe for injection (Terumo Medical Products, Elkton, MD). The artery blood in the syringe is incubated at 30∘C. for 2 days to form a blood clot. After that, 0.1 mL of physiological saline is added to the syringe for injection and passed through a 26-gauge injection needle (Terumo Medical Products, Elkton, MD) twice so that the blood clot is crushed.

Rats in which a cerebral embolic stroke is induced are anesthetized with 1.0% halothane under spontaneous respiration. The neck of the rats is subjected to a midline incision and external carotid artery, superior thyroid artery, occipital artery and pterygopalatine artery are cauterized with a bipolar coagulator (T-45; Keisei Medical Industrial Co. Ltd, Tokyo, Japan). Cerebral embolism is induced by injecting 0.1 mL of the crushed blood clot into the internal carotid.

Evaluation of the formation of a cerebral embolism is carried out using a laser Doppler flowmetry (FloC1; Omegawave, Tokyo, Japan). A decrease in cerebral blood flow to a level of 30% or less is taken as a positive evidence of embolism formation. The cerebral blood flow is monitored for 30 minutes after infusion of the blood clot and blood flow is monitored as remaining at 50% or less of the flow prior to the injection of the blood clot. After that, a cannula (PE50) for administration of the medicament is secured in the jugular vein and the animals are woken.

The rats that have successfully formed a cerebral embolism are divided into four groups. The first group of rats is given a saline injection every other day. Groups 2-4 are given 0.125mg/m² injection of TPA every other day for four weeks. Group 2 is then sacrificed. Groups 3-4 are given a further 0.156 mg/m² of TPA every other day for two weeks and then Group 3 is sacrificed. Group 4 is given 0.18775 mg/m² of TPA every other day for three weeks and then sacrificed.

The brains are excised after the animals are sacrificed and sliced in ten sections at 1 mm intervals using a Mclwain tissue chopper (Mickle Laboratory Engineering, U.K.) and are stained by dipping for 20 minutes in a 2% TTC (2,3,5-triphenyltetrazolium chloride; Tokyo Kasei) at 37∘C. Images of the TTC-stained slices are uploaded into a computer using a digital camera (HC-2500; Fuji PhotoFilm) and Phatograb-2500 (Fuji Photo Film) and infarct volume is calculated using Mac Scope (Mitani, Japan). Infarct volume is given by a mean value ± standard error. With regard to the statistical test of the result of the infarct volume, the evaluation is done by carrying out a Dunnett's test for control group and for each of the TPA -administered groups as compared with the control group and then by carrying out the t-test for the TPA-administered group.

Neurological symptoms are observed daily until sacrifice and the rats are evaluated according to three tests: (1) Rats are held gently by the tail, suspended one meter above the floor, and observed for forelimb flexion; (2) Rats are placed on a large sheet of soft, plastic coated paper that could be gripped firmly by their claws. With the tail held by hand, gentle lateral pressure is applied behind the rat's shoulder until the forelimbs slid several inches; (3) Rats are allowed to move about freely and are observed for circling behavior. Scoring of the neurological symptoms is carried out according to the scale developed by Bederson et al. (1986) as follows: 0: no observable deficit; 1: forelimb flexion; 2: decreased resistance to lateral push without circling; 3: same behavior as grade 2, with circling.

Neurological symptoms are evaluated using a Steel's test for the control group and for each of the TPA administered groups as compared with the control group and then by carrying out a Wilcoxon test for the TPA administered group. In any of the tests, the value where p<0.05 is defined to be statistically significant.

### Example VIII

### Effectiveness of TPA in the Treatment of Stroke Using A Permanent Middle Cerebral Artery Occlusion Model

Male Wistar rats (250-320 g) are used for this study. Animals are anesthetized with Isoflurane (3% induction, 1-2% maintenance). Anesthesia is monitored by toe pinch. Aseptic technique is used for all procedures during this study. The surgical site is clipped and cleaned with alcohol and surgical scrub. The animal is placed on a warm water heating pad to maintain body temperature. A paramedian incision is made on the neck over the carotid artery. The tissue is bluntly dissected away to reveal the carotid artery and the bifurcation. Sutures are placed around the proximal portion or the common carotid and the external carotid arteries. These sutures are tied off. An incision is made in the common carotid, distal to the ligation. A pre-prepared filament (4-0 monofilament suture or like material) is placed in the carotid and advanced into the internal carotid artery. The filament is advanced about 20 mm past the carotid bifurcation until slight resistance is felt as it wedges in the middle cerebral artery. Care must be taken to not rupture the artery upon insertion of the filament. The filament is tied in place and the skin incision closed. The animal is evaluated when awake for successful occlusion using the Bederson scale. (See Bederson et al., (1986) Stroke, 17:1304-1308.) Body temperature is taken every 15 minutes to maintain normothermia. Animals that have undergone the middle cerebral artery occlusion procedure may have difficulty in thermoregulation for a few hours after surgery Animals are placed in a cooling or heating box as determined by their temperature. Body temperature is maintained at 37.5∘ C. Animals are monitored for 6 hours following middle cerebral artery and are then placed in cages overnight.

The rats are divided into four groups. The first group of rats is given saline injections every other day. Groups 2-4 are given 0.125mg/m² injection of TPA every other day for four weeks. Group 2 is then sacrificed. Groups 3-4 are given a further 0.156mg/m² of TPA every other day for two weeks and then Group 3 is sacrificed. Group 4 is given 0.18775mg/m² of TPA every other day for three weeks and then sacrificed.

The brains are excised after the animals are sacrificed and sliced in ten sections at 1 mm intervals using a McIwain tissue chopper (Mickle Laboratory Engineering, U.K.) and are stained by dipping for 20 minutes in a 2% TTC (2,3,5-triphenyltetrazolium chloride; Tokyo Kasei) at 37∘C. Images of the TTC-stained slices are uploaded into a computer using a digital camera (HC-2500; Fuji PhotoFilm) and Phatograb-2500 (Fuji Photo Film). Brain slices are photographed and analyzed for infarct size, infarct volume, penumbra, and edema.

Neurological symptoms are observed daily until sacrifice. Neurological symptoms are observed daily until sacrifice and the rats are evaluated according to three tests. (1) Rats are held gently by the tail, suspended one meter above the floor, and observed for forelimb flexion. (2) Rats are placed on a large sheet of soft, plastic coated paper that could be gripped firmly by their claws. With the tail held by hand, gentle lateral pressure is applied behind the rat's shoulder until the forelimbs slid several inches. (3) Rats are allowed to move about freely and are observed for circling behavior. Scoring of the neurological symptoms is carried out according to the scale developed by Bederson et al. (1986) as follows: 0: no observable deficit; 1: forelimb flexion; 2: decreased resistance to lateral push without circling; 3: same behavior as grade 2, with circling.

Neurological symptoms are evaluated using a Steel's test for the control group and for each of the TPA administered groups as compared with the control group and then by carrying out a Wilcoxon test for the TPA administered group. In any of the tests, the value where p<0.05 is defined to be statistically significant.

### Example IX

### Effectiveness of TPA in the Treatment of Stroke Using A Temporary Middle Cerebral Artery Occlusion Model

Male C57B16 mice (25-30 g) are used in this study. Mice are anesthetized with Isoflurane (3% induction, 1-2% maintenance). The surgical site is clipped and cleaned with alcohol and surgical scrub. A midline neck incision is made over the carotid artery and the artery is dissected to its bifurcation. A monofilament suture is introduced into the internal carotid artery and advanced until it lodges in the middle cerebral artery. The suture is tied in placed and the incision is closed. Two hours after occlusion the mice will be re-anesthetized and the suture will be removed from the MCA. Body temperature is maintained by use of a heating pad both during and after surgery. Animals are monitored for 4 hours following middle cerebral artery occlusion.

The rats are divided into four groups. The first group of rats is given saline injections every other day. Groups 2-4 are given 0.125mg/m² injection of TPA every other day for four weeks. Group 2 is then sacrificed. Groups 3-4 are given a further 0.156mg/m²of TPA every other day for two weeks and then Group 3 is sacrificed. Group 4 is given 0.18775mg/m² of TPA every other day for three weeks and then sacrificed.

The brains are excised after the animals are sacrificed and sliced in ten sections at 1 mm intervals using a McIwain tissue chopper (Mickle Laboratory Engineering, U.K.) and are stained by dipping for 20 minutes in a 2% TTC (2,3,5-triphenyltetrazolium chloride; Tokyo Kasei) at 37∘C. Images of the TTC-stained slices are uploaded into a computer using a digital camera (HC-2500; Fuji PhotoFilm) and Phatograb-2500 (Fuji Photo Film). Brain slices are photographed and analyzed for infarct size, infarct volume, penumbra, and edema.

Neurological symptoms are observed daily until sacrifice and the rats are evaluated according to three tests. (1) Rats are held gently by the tail, suspended one meter above the floor, and observed for forelimb flexion. (2) Rats are placed on a large sheet of soft, plastic coated paper that could be gripped firmly by their claws. With the tail held by hand, gentle lateral pressure is applied behind the rat's shoulder until the forelimbs slid several inches. (3) Rats are allowed to move about freely and are observed for circling behavior. Scoring of the neurological symptoms is carried out according to the scale developed by Bederson et al. (1986) as follows: 0: no observable deficit; 1: forelimb flexion; 2: decreased resistance to lateral push without circling; 3: same behavior as grade 2, with circling.

Neurological symptoms are evaluated using a Steel's test for the control group and for each of the TPA administered groups as compared with the control group and then by carrying out a Wilcoxon test for the TPA administered group. In any of the tests, the value where p<0.05 is defined to be statistically significant.

### Example X

### Clinical Effectiveness of the Use of TPA to Treat Stroke

Males and Females between the ages of 30-72 years who suffered a stroke less than one month previously are recruited for participation in a ten week trial of TPA.

Recruited individuals sign an informed consent form and are evaluated using computed tomography (CT), physical and neurological tests, neurological check, sedation level, National Institute of Health Stroke Survey (NIHSS), 12-lead electrocardiogram, telemetry of electrocardiogram, pulse oxygen measurement, vital sign, body weight, background of the patient, test on pregnancy, measurement of medicament in urine, hematological test, coagulation panel, general clinical test, urine test. Clinical Laboratory Testing includes a Complete Metabolic Panel (Na, K, Cl, CO2, Glu, BUN, Cr, Ca, TP, Alb, TBili, AP, AST, ALT), Hematology CBC (Hgb, Hct, RBC, WBC, Plt, Diff), and Serum hCG for all females.

Individuals are administered 0.125mg/m² of TPA or placebo every other day for four weeks, then 1.25 × 0.125mg/m² or placebo every other day for weeks five and six and 1.5 × 0.125mg/m² or placebo every other day for weeks seven to nine. Individuals are monitored during and for two hours after administration of TPA or placebo.

At weeks five and week ten, subjects are evaluated using the NIHSS (NIH Stroke Scale), the Barthel ADL index (Granger, 1979), and a modified Rankin scale (Farrell, 1991),

Efficacy is determined by measuring the change from baseline in the NIHSS in individuals treated with TPA in comparison to placebo. Secondary efficacy variables are the Barthel ADL index and a modified Rankin scale. Safety measures are collected and evaluated through the trial, specifically measuring changes from baseline visit to week 5. These measures include adverse event reports, physical examinations, vital signs, weight measurements, ECGs, clinical laboratory test results, and vital signs as well as scores for suicidal behaviors and/or ideation. Adverse events are any untoward medical event occurring in a subject administered study drug, irrespective of whether it has a causal relationship to the study drug. An adverse event can therefore be any unfavorable or unintended sign (including abnormal laboratory findings, for example), symptom, or disorder temporarily associated with study drug, whether or not considered related to the study drug.

Subjects are considered to have completed the study if they complete all of the visits. They may be terminated from the study if they fail to meet inclusion/exclusion criteria; suffer from an adverse event, have an insufficient therapeutic response, withdraw their consent, violate the protocol, stop coming, or die.

### References

Altuwaijri S, Lin H K, Chuang K H, Lin W J, Yeh S, Hanchett L A, Rahman M M, Kang H Y, Tsai M Y, Zhang Y, Yang L, and Chang C. Interruption of nuclear factor kappaB signaling by the androgen receptor facilitates 12-O-tetradecanoylphorbolacetate-induced apoptosis in androgen-sensitive prostate cancer LNCaP cells. Cancer Res 2003; 63: 7106-12.

Ando I., Crawfor D. H. et al. Phorbol ester-induced expression and function of the interleukin 2 receptor in human B lymphocytes. Eur J Immunol. 15(4), 341-4 (1985).

Aye M. T., Dunne J.V. Opposing effects of 12-O-tetradecanoylphorbol 13-acetate on human myeloid and lymphoid cell proliferation. J Cell Physiol. 114(2), 209-14 (1983).

Bederson JB, Pitts LH, Tsuji M, Nishimura MC, Davis RL, Bartkowski H. Rat middle cerebral artery occlusion: evaluation of the model and development of a neurologic examination. Stroke. 1986; 17: 472-476.

Boutwell R.K. Biochemical mechanism of tumor promotion, in mechanisms of tumor promotion and co-carcinogenesis. Eds. Slaga, T.J., Sivak, A.J. and Boutwell, R.K. Raven, New York, 49-58 (1978).

Boutwell R.K. The function and mechanism of promoters of carcinogenesis. CRC Crit. Rev. Toxicol 2, 419-443 (1974).

Brose N, Rosenmund C. Move over protein kinase C, you've got company: alternative effectors of diacylglycerol and phorbol esters. JCell Sci;115:4399-411 (2002).

Cancer Chemother Pharmacol. Jun;57(6):789-95 (2006).

Cui X X, Chang R L, Zheng X, Woodward D, Strair R, and Conney A H. A sensitive bioassay for measuring blood levels of 12-O-tetradecanoylphorbol-13-acetate (TPA) in patients: preliminary pharmacokinetic studies. Oncol Res 2002; 13: 169-74.

Deegan M. J., Maeda k. Differentiation of chronic lymphocytic leukemia cells after in vitro treatment with Epstein-Barr virus or phorbol ester. Immunologic and morphologic studies. Am J Hermatol. 17(4), 335-47 (1984).

Falcioni F., Rautmann A. et al. Influence of TPA (12-O-tetradodecanoyl-phorbol-13-acetate) on human B lymphocte function. Clin Exp Immunol. 62(3), 163-2 (1985).

Farrell B, Godwin J, Richards S, Warlow C, et al. (1991). "The United Kingdom transient ischaemic attack (UK-TIA) aspirin trial: final results." J Neurol Neurosurg Psychiatry 54 (12): 1044-1054.

Forbes I. J., Zalewski P. D., Letarte M. Human B-lymphocyte maturation sequence revealed by TPA-induced differentiation of leukaemi cells. Immunobiology 163(1), 1-6 (1982).

Gunjan Goel, Harinder P. S. Makkar, George Francis, and Klaus Becker. Phorbol Esters: Structure, Biological Activity, and Toxicity in Animals. International Journal of Toxicology, 26:279-288, 2007.Gogusev J., Barbey S., Nezelof C. Regulation of TNF-alpha and IL-1 gene expression during TPA-induced differentiation of "Malignant histiocyosis" DEL cell line t(5:6) (q35:P21). Anticancer Res. 16(1), 455-60 (1996).

Granger CV, Devis LS, Peters MC, Sherwood CC, Barrett JE. Stroke rehabilitation: analysis of repeated Barthel Index measures. Arch Phys Med Rehabil. 1979;60:14-17.

Han Z T, Zhu X X, Yang R Y, Sun J Z, Tian G F, Liu X J, Cao G S, Newmark H L, Conney A H, and Chang R L. Effect of intravenous infusions of 12-0-tetradecanoylphorbol-13-acetate (TPA) in patients with myelocytic leukemia: preliminary studies on therapeutic efficacy and toxicity. Proc Natl Acad Sci USA 1998; 95: 5357-61.

Han Z. T., Tong Y. K., He L. M., Zhang Y., Sun J. Z., Wang T. Y., Zhang H., Cui Y. L., Newmark H. L., Conney A. H., Chang R. L. 12-O-Tetradecanoyl-phorbol-13-acetate (TPA) -induced increase in depressed white blood cell counts in patients treated with cytotoxic cancer chemotherapeutic drugs. Proc. Natl. Acad. Sci. 95, 5363-5365 (1998).

Han Z.T., Zhu X. X., Yang R. Y., Sun J. Z., Tian G. F., Liu X. J., Cao G. S., NewMark H. L., Conney A. H., and Chang R. L. Effect of intravenous infusion of 12-0-tetradecanoyl-phorbol-13-acetate (TPA) in patients with myelocytic leukemia: preliminary studies on therapeutic efficacy and toxicity. Pro. Natl. Acad. Sci. 95, 5357-5361 (1998).

Harada S. et al.: Tumor Promoter, TPA, Enhances Replication of HTLV-III/LAV. Virology 154, 249-258 (1986).

Hecker E. In handbuch der allgemeinen patholgie, ed. Grundmann, E. (Springer-Verlag, Berlin-Heideiberg, Vol. IV 16, 651-676 (1975).

Hecker E. Structure -activity relationships in deterpene esters irritant and co-carcinogenic to mouse skin, in mechanisms of tumor promotion and co-carcinogenesis. Eds. Slaga, T. J., Sevak, A. j. and Boutwell, R.K. Raven, New York, 11-49 (1978).

Hofmann J. The potential for isoenzyme-selective modulation of protein kinase C. FASEB J. 11, 649-669 (1997).

Huberman E., Callaham M. F. Induction of terminal differentiation in human promyelocytic leukemia cells by tumor-promoting agents. Proc. Natl. Acad. Sci.76, 1293-1297 (1979).

Hunter T. Signaling 2000 and beyond. Cell 100, 113-117 (2000).

Kassel O, Sancono A, Kratzschmar J, Kreft B, Stassen M, and Cato A C. Glucocorticoids inhibit MAP kinase via increased expression and decreased degradation of MKP-1. Embo J 2001; 20: 7108-16.

Kazanietz M.G. Eyes Wide Shut: protein kinase C isoenzymes are not the only receptors for the phorbol ester tumor promoters. Mol. Carcinog. 28, 5-12 (2000).

Keoffler H. P., Bar-Eli M., Territo M. C. Phorbol ester effect on differentiation of human myeloid leukemia cells lines blocked at different stages of maturation. Cancer Res. 41, 919-926 (1981).

Kim S C, Hahn J S, Min Y H, Yoo N C, Ko Y W, and Lee W J. Constitutive activation of extracellular signal-regulated kinase in human acute leukemias: combined role of activation of MEK, hyperexpression of extracellular signal-regulated kinase, and downregulation of a phosphatase, PAC1. Blood 1999; 93: 3893-9.

Kiyoi H, Naoe T, Nakano Y, Yokota S, Minami S, Miyawaki S, Asou N, Kuriyama K, Jinnai I, Shimazaki C, Akiyama H, Saito K, Oh H, Motoji T, Omoto E, Saito H, Ohno R, and Ueda R. Prognostic implication of FLT3 and N-RAS gene mutations in acute myeloid leukemia. Blood 1999; 93: 3074-80.

Kobayashi M., Okada N. et al. Intracellular interleukin-1 alpha production in human gingival fibroblasts is differentially regulated by various cytokines. J Dent Res. 78(4), 840-9 (1999).

Kudo M., Aoyama A., Ichimori S. and Fulcunaga N. An animal model of cerebral infarction: homologous blood clot emboli in rats. Stroke 13: 505-508 (1982)

Lebien T. W., Bollum F. J. et al. Phorbol ester-induced differentiation of a non-T, non-B leudemic cell line: model for human lymphoid progenitor cell development. J Immunol. 128(3), 1316-20 (1982).

MD Iqbal Hossain Chowdhury et al. The Phorbol Ester TPA Strongly Inhibits HIV-1 Induced Syncytia Formation but Enhances Virus Production: possible involvement of protein kinase C pathway. Virology 176, 126-132, (1990).

Meinhardt G., Roth J., Hass R. Activation of protein kinase C relays distinct signaling pathways in the same cell type: differentiation and caspase-mediated apoptosis. Cell Death Differ. 7, 795-803 (2000).

Milella M, Komblau S M, Estrov Z, Carter B Z, Lapillonne H, Harris D, Konopleva M, Zhao S, Estey E, and Andreeff M. Therapeutic targeting of the MEK/MAPK signal transduction module in acute myeloid leukemia. J Clin Invest 2001; 108: 851-9.

Mochty-Rosen D., Kauvar L. M. Modulating protein kinase C signal transduction. Adv. Pharmacol. 44, 91-145 (1998).

Morgan M A, Dolp O, and Reuter C W. Cell-cycle-dependent activation of mitogen-activated protein kinase kinase (MEK-1/2) in myeloid leukemia cell lines and induction of growth inhibition and apoptosis by inhibitors ofRAS signaling. Blood 2001; 97: 1823-34.

Nagasawa K., Chechgik B. E. et al. Modulation of human T-cell differentiation markers by 12-O-tetradecanoylphorbal-13-acetate. Thymus. 3(4-5), 307-18, (1981).

Nakao Y., Matsuda S. et al. Paradoxical anti-leukemic effects of plant-derived tumor promoters on a human thymic lymphoblast cell line. Int J Cancer 30(6), 687-95 (1982).

Nakao Y., Matsuda S. et al. Phorbol ester-induced differentiation of human T-lymphoblastic cell line HPB-ALL. Cancer Res. 42(9), 33843-50 (1982).

Newton A.C. Protein kinase C: structure, function and regulation. J. Biol. Chem. 270, 28495-28499 (1995).

Palombella V J, Rando O J, Goldberg A L, and Maniatis T. The ubiquitin-proteasome pathway is required for processing the NF-kappa B1 precursor protein and the activation of NF-kappa B. Cell 1994; 78: 773-85.

Platanias L C. Map kinase signaling pathways and hematologic malignancies. Blood 2003; 101: 4667-79.

Rovera G., Santoli D., Damsky C. Human promyelocytic cells in culture differentiate into macrophage-like cells treated with a phorbol diester. Pro. Natl. Acad. Sci. 7, 2779-2783 (1979).

YIP, Y.K. et al. Stimulation of human gamma interferon production by diterpene esters. Infection and Immunity 34(1) 131-139 (1981).

Zhao J., Sharma Y., Agarwal R. Significant inhibition by the flavonoid antioxidant silymarin against 12-O-tetradecanoylphorbol 13-acetate-caused modulation of antioxidant and inflammatory enzymes and cyclooxygenase2 and interleukin-I alpha expression in SENCAR mouse epidermis: implications in the prevention of stage 1 tumor promotion. Mol Carcinog. 26(4), 321-33 (1999).

## Claims

1. A phorbol ester of Formula I, or a pharmaceutically-acceptable salt or enantiomer thereof for use in a method for preventing or treating one or more of the effects of stroke in a mammalian subject or a composition comprising a phorbol ester of Formula I, or a pharmaceutically-acceptable salt or enantiomer thereof for use in a method for the prevention or treatment of one or more effects of stroke in a mammalian subject; 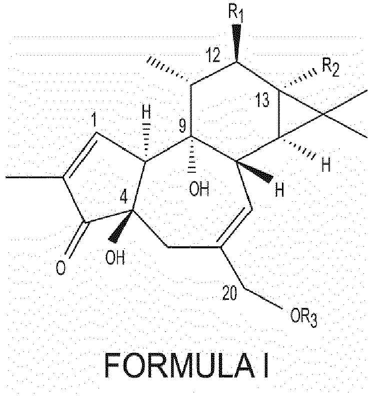
wherein R₁ and R₂ are selected from the group consisting of hydrogen, hydroxyl, 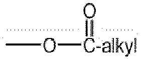 wherein the alkyl group contains 1 to 15 carbon atoms, 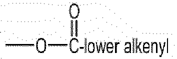 wherein the lower alkenyl group contains 1 to 7 carbon atoms, 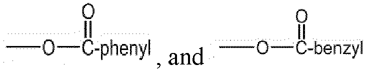 ; and R₃ is hydrogen, or 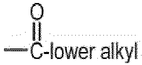 wherein the lower alkyl group contains 1 to 7 carbon atoms;
wherein the one or more of the effects of stroke are paralysis, spatial impairment, impaired judgment, left-sided neglect, memory loss, aphasia, coordination and balance problems, nausea, vomiting, cognitive impairment, perception impairment, orientation impairment, homonymous hemianopsia, or impulsivity; and
wherein the phorbol ester of Formula I is in a unit dosage amount from about 10 µg to about 1500 µg.

2. The phorbol ester or composition of claim 1, for use of claim 1, wherein R₁ or R₂ is 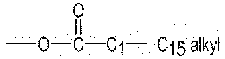 the remaining R₁ or R₂ is 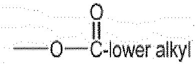 wherein the lower alkyl group contains 1 to 7 carbon atoms, and R₃ is hydrogen.

3. The phorbol ester or composition of claim 1, for use of claim 1, wherein the phorbol ester is phorbol 13-butyrate, phorbol 12-decanoate, phorbol 13-decanoate, phorbol 12,13-diacetate, phorbol 13,20-diacetate, phorbol 12,13-dibenzoate, phorbol 12,13-dibutyrate, phorbol 12,13-didecanoate, phorbol 12,13-dihexanoate, phorbol 12,13-dipropionate, phorbol 12-myristate, phorbol 13-myristate, phorbol 12,13,20-triacetate, 12-deoxyphorbol 13-angelate, 12-deoxyphorbol 13-angelate 20-acetate, 12-deoxyphorbol 13-isobutyrate, 12-deoxyphorbol 13-isobutyrate-20-acetate, 12-deoxyphorbol 13-phenylacetate, 12-deoxyphorbol 13-phenylacetate 20-acetate, 12-deoxyphorbol 13-tetradecanoate, phorbol 12-tigliate 13-decanoate, 12-deoxyphorbol 13-acetate, phorbol 12-acetate, or phorbol 13-acetate.

4. The phorbol ester or composition of claim 1, for use of claim 1, wherein the method or composition further comprises administering at least one secondary or adjunctive therapeutic agent that is effective in a combinatorial formulation or coordinate treatment regimen with said phorbol ester of Formula I to treat or prevent effects of stroke in said subject.

5. The phorbol ester or composition of claim 4, for use of claim 4, wherein the at least one secondary or adjunctive therapeutic agent is intended to be administered to said subject in a coordinate administration protocol, simultaneously with, prior to, or after, administration of said phorbol ester to said subject.

6. The phorbol ester or composition of claim 4, for use of claim 4, wherein the at least one secondary or adjunctive therapeutic agent is tissue plasminogen activator, an anticoagulant, a statin, fibrate, angiotensin II receptor blockers, angiotensin-converting enzyme inhibitor, beta-blocker, antiplatelet agent, calcium channel blocker, or diuretic.

7. The phorbol ester or composition of claim 1, for use of claim 1, wherein the method further comprises surgical intervention in combination with phorbol ester of Formula I to treat or prevent effects of stroke in said subject.

8. The phorbol ester or composition of claim 7, for use of claim 7, wherein the surgical intervention is a carotid endarterectomy, angioplasty, stent placement, craniotomy, insertion of a pacemaker, implantation of a defibrillator, replacement of valves, coronary artery bypass, heart transplantation, endovascular coil embolization, or patent foramen ovale closure.

9. The phorbol ester or composition of any one of claims 1 to 8, for use of any one of claims 1 to 8, wherein the mammalian subject is a mammalian subject who has suffered a stroke.

10. A phorbol ester of Formula I, or a pharmaceutically-acceptable salt or enantiomer thereof for use in a method for preventing or treating one or more symptoms of a stroke in a mammalian subject, or a composition comprising a phorbol ester of Formula I, or a pharmaceutically-acceptable salt or enantiomer thereof for use in a method for preventing or treating one or more symptoms of a stroke in a mammalian subject; 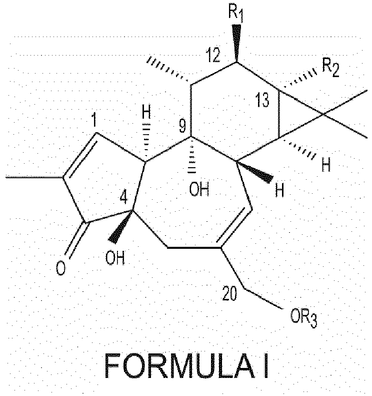
wherein R₁ and R₂ are selected from the group consisting of hydrogen, hydroxyl, 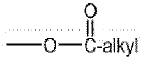 wherein the alkyl contains 1 to 15 carbon atoms, 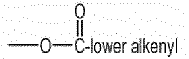 wherein the lower alkenyl group contains 1 to 7 carbon atoms, 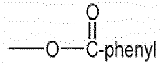 , and 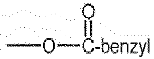 ; and R₃ is hydrogen and 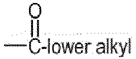 wherein the lower alkyl group contain 1 to 7 carbon atoms;
wherein the one or more symptoms of stroke is sudden numbness or weakness on one side of the body, sudden confusion or trouble speaking or understanding speech, sudden trouble with walking, dizziness or loss of balance or coordination; and
wherein the phorbol ester of Formula I is in a unit dosage amount from about 10 µg to about 1500 µg.

11. The phorbol ester or composition of claim 10, for use of claim 10, wherein R₁ or R₂ is 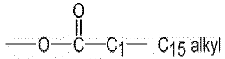 and the remaining R₁ or R₂ is 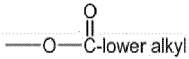 wherein the lower alkyl group contains 1 to 7 carbon atoms, and R₃ is hydrogen.

12. The phorbol ester or composition of claim 10, for use of claim 10, wherein the phorbol ester is phorbol 13-butyrate, phorbol 12-decanoate, phorbol 13-decanoate, phorbol 12,13-diacetate, phorbol 13,20-diacetate, phorbol 12,13-dibenzoate, phorbol 12,13-dibutyrate, phorbol 12,13-didecanoate, phorbol 12,13-dihexanoate, phorbol 12,13-dipropionate, phorbol 12-myristate, phorbol 13-myristate, phorbol 12,13,20-triacetate, 12-deoxyphorbol 13-angelate, 12-deoxyphorbol 13-angelate 20-acetate, 12-deoxyphorbol 13-isobutyrate, 12-deoxyphorbol 13-isobutyrate-20-acetate, 12-deoxyphorbol 13-phenylacetate, 12-deoxyphorbol 13-phenylacetate 20-acetate, 12-deoxyphorbol 13-tetradecanoate, phorbol 12-tigliate 13-decanoate, 12-deoxyphorbol 13-acetate, phorbol 12-acetate, or phorbol 13-acetate.

13. The phorbol ester or composition of claim 10, for use of claim 10, further comprising administering at least one secondary or adjunctive therapeutic agent that is effective in a combinatorial formulation or coordinate treatment regimen with said phorbol ester of Formula I to treat or prevent effects of stroke in said subject, wherein the secondary or adjunctive therapeutic agent is administered simultaneously with, prior to, or after, administration of said phorbol ester of Formula I.

14. The phorbol ester or composition of claim 13, for use of claim 13, wherein the at least one secondary or adjunctive therapeutic agent is an anticoagulant, a statin, fibrate, angiotensin II receptor blockers, antiplatelet agent, angiotensin-converting enzyme inhibitor, beta-blocker, calcium channel blocker, or diuretic.

15. The phorbol ester or composition of claim 1, for use of claim 1 or phorbol ester of claim 10, for use of claim 10, wherein the phorbol ester is 12-O-tetradecanoylphorbol-13-acetate (TPA).

## Patentansprüche

1. Phorbolester der Formel I, oder ein pharmazeutisch verträgliches Salz oder Enantiomer davon, zur Verwendung in einem Verfahren zur Verhütung oder Behandlung einer oder mehrerer der Auswirkungen eines Schlaganfalls bei einem Säugerindividuum, oder Zusammensetzung, umfassend einen Phorbolester der Formel I, oder ein pharmazeutisch verträgliches Salz oder Enantiomer davon, zur Verwendung in einem Verfahren zur Verhütung oder Behandlung einer oder mehrerer Auswirkungen eines Schlaganfalls bei einem Säugerindividuum; 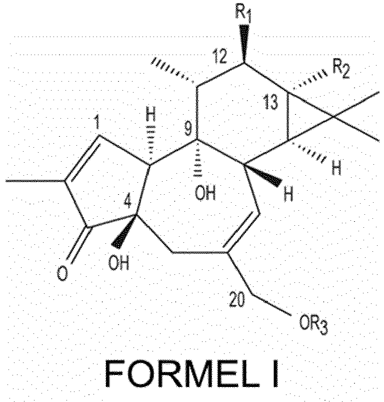
wobei Ri und R₂ aus der Gruppe ausgewählt sind, die besteht aus Wasserstoff, Hydroxyl, 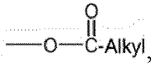 wobei die Alkylgruppe 1 bis 15 Kohlenstoffatome enthält, 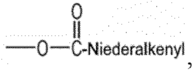 wobei die Niederalkenylgruppe 1 bis 7 Kohlenstoffatome enthält, 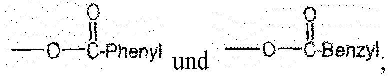 und R₃ Wasserstoff oder 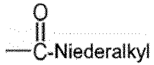 ist, wobei die Niederalkylgruppe 1 bis 7 Kohlenstoffatome enthält;
wobei die eine oder mehreren der Auswirkungen eines Schlaganfalls Paralyse, räumliche Beeinträchtigung, beeinträchtigtes Urteilsvermögen, linksseitiger Neglect, Gedächtnisverlust, Aphasie, Koordinations- und Gleichgewichtsprobleme, Übelkeit, Erbrechen, kognitive Beeinträchtigung, Wahrnehmungsbeeinträchtigung, Orientierungsbeeinträchtigung, homonyme Hemianopsie oder Impulsivität sind; und
wobei der Phorbolester der Formel I in einer Dosierungsmengeneinheit von ungefähr 10 µg bis ungefähr 1500 µg vorliegt.

2. Phorbolester oder Zusammensetzung nach Anspruch 1 zur Verwendung nach Anspruch 1, wobei R₁ oder R₂ 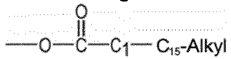 ist, das verbleibende R₁ oder R₂ 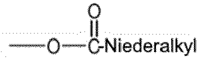 ist wobei die Niederalkylgruppe 1 bis 7 Kohlenstoffatome enthält und R₃ Wasserstoff ist.

3. Phorbolester oder Zusammensetzung nach Anspruch 1, zur Verwendung nach Anspruch 1, wobei der Phorbolester Phorbol-13-butyrat, Phorbol-12-decanoat, Phorbol-13-decanoat, Phorbol-12,13-diacetat, Phorbol-13,20-diacetat, Phorbol-12,13-dibenzoat, Phorbol-12,13-dibutyrat, Phorbol-12,13-didecanoat, Phorbol-12,13-dihexanoat, Phorbol-12,13-dipropionat, Phorbol-12-myristat, Phorbol-13-myristat, Phorbol-12,13,20-triacetat, 12-Desoxyphorbol-13-angelat, 12-Desoxyphorbol-13-angelat-20-acetat, 12-Desoxyphorbol-13-isobutyrat, 12-Desoxyphorbol-13-isobutyrat-20-acetat, 12-Desoxyphorbol-13-phenylacetat, 12-Desoxyphorbol-13-phenylacetat-20-acetat, 12-Desoxyphorbol-13-tetradecanoat, Phorbol-12-tigliat-13-decanoat, 12-Desoxyphorbol-13-acetat, Phorbol-12-acetat oder Phorbol-13-acetat ist.

4. Phorbolester oder Zusammensetzung nach Anspruch 1, zur Verwendung nach Anspruch 1, wobei das Verfahren oder die Zusammensetzung ferner Verabreichen mindestens eines sekundären oder begleitenden therapeutischen Mittels umfasst, das in einer kombinatorischen Formulierung oder einem Regime koordinierter Behandlung mit dem Phorbolester der Formel I wirksam ist, um Auswirkungen eines Schlaganfalls bei dem Individuum zu behandeln oder zu verhüten.

5. Phorbolester oder Zusammensetzung nach Anspruch 4, zur Verwendung nach Anspruch 4, wobei das mindestens eine sekundäre oder begleitende therapeutische Mittel dazu bestimmt ist, dem Individuum in einem Protokoll koordinierter Verabreichung verabreicht zu werden, gleichzeitig mit, vor, oder nach, Verabreichung des Phorbolesters an das Individuum.

6. Phorbolester oder Zusammensetzung nach Anspruch 4, zur Verwendung nach Anspruch 4, wobei das mindestens eine sekundäre oder begleitende therapeutische Mittel ein Gewebeplasminogenaktivator, Antikoagulans, Statin, Fibrat, Angiotensin-II-Rezeptor-Blocker, ein Hemmer des Angiotensin konvertierenden Enzyms, Beta-Blocker, Thrombozytenaggregationshemmer, Kalziumkanalblocker oder Diuretikum ist.

7. Phorbolester oder Zusammensetzung nach Anspruch 1, zur Verwendung nach Anspruch 1, wobei das Verfahren ferner einen chirurgischen Eingriff in Kombination mit Phorbolester der Formel I umfasst, um Auswirkungen eines Schlaganfalls bei dem Individuum zu behandeln oder zu verhüten.

8. Phorbolester oder Zusammensetzung nach Anspruch 7, zur Verwendung nach Anspruch 7, wobei der chirurgische Eingriff eine Carotis-Endarteriektomie, Angioplastie, Stentplatzierung, Kraniotomie, Einsetzung eines Schrittmachers, Implantierung eines Defibrillators, Ersetzung von Klappen, Koronararterienbypass-OP, Herztransplantation, endovaskuläre Embolisation bzw. Coiling oder Schließung eines offenen Foramen ovale ist.

9. Phorbolester oder Zusammensetzung nach einem der Ansprüche 1 bis 8, zur Verwendung nach einem der Ansprüche 1 bis 8, wobei das Säugerindividuum ein Säugerindividuum ist, das einen Schlaganfall erlitten hat.

10. Phorbolester der Formel I, oder ein pharmazeutisch verträgliches Salz oder Enantiomer davon, zur Verwendung in einem Verfahren zur Verhütung oder Behandlung eines oder mehrerer Symptome eines Schlaganfalls bei einem Säugerindividuum, oder Zusammensetzung, umfassend einen Phorbolester der Formel I, oder ein pharmazeutisch verträgliches Salz oder Enantiomer davon, zur Verwendung in einem Verfahren zur Verhütung oder Behandlung eines oder mehrerer Symptome eines Schlaganfalls bei einem Säugerindividuum; 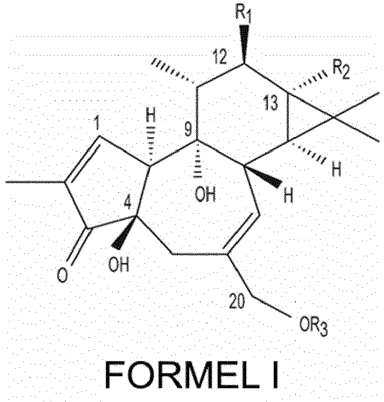
wobei R₁ und R₂ aus der Gruppe ausgewählt sind, die besteht aus Wasserstoff, Hydroxyl, 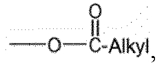 wobei das Alkyl 1 bis 15 Kohlenstoffatome enthält, 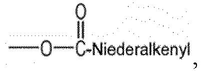 wobei die Niederalkenylgruppe 1 bis 7 Kohlenstoffatome enthält, 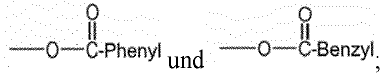 und R₃ Wasserstoff und 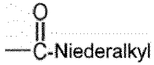 ist, wobei die Niederalkylgruppe 1 bis 7 Kohlenstoffatome enthält;
wobei das eine oder die mehreren Symptome eines Schlaganfalls plötzliche Taubheit oder Schwäche auf einer Seite des Körpers, plötzliche Verwirrtheit oder Sprachoder Sprachverständnisstörung, plötzliche/-r Gangunsicherheit, Schwindel oder Gleichgewichts- oder Koordinationsverlust sind; und
wobei der Phorbolester der Formel I in einer Dosierungsmengeneinheit von ungefähr 10 µg bis ungefähr 1500 µg vorliegt.

11. Phorbolester oder Zusammensetzung nach Anspruch 10, zur Verwendung nach Anspruch 10, wobei R₁ oder R₂ 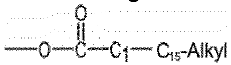 ist und das verbleibende R₁ oder R₂ 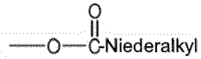 ist, wobei die Niederalkylgruppe 1 bis 7 Kohlenstoffatome enthält, und R₃ Wasserstoff ist.

12. Phorbolester oder Zusammensetzung nach Anspruch 10, zur Verwendung nach Anspruch 10, wobei der Phorbolester Phorbol-13-butyrat, Phorbol-12-decanoat, Phorbol-13-decanoat, Phorbol-12,13-diacetat, Phorbol-13,20-diacetat, Phorbol-12,13-dibenzoat, Phorbol-12,13-dibutyrat, Phorbol-12,13-didecanoat, Phorbol-12,13-dihexanoat, Phorbol-12,13-dipropionat, Phorbol-12-myristat, Phorbol-13-myristat, Phorbol-12,13,20-triacetat, 12-Desoxyphorbol-13-angelat, 12-Desoxyphorbol-13-angelat-20-acetat, 12-Desoxyphorbol-13-isobutyrat, 12-Desoxyphorbol-13-isobutyrat-20-acetat, 12-Desoxyphorbol-13-phenylacetat, 12-Desoxyphorbol-13-phenylacetat-20-acetat, 12-Desoxyphorbol-13-tetradecanoat, Phorbol-12-tigliat-13-decanoat, 12-Desoxyphorbol-13-acetat, Phorbol-12-acetat oder Phorbol-13-acetat ist.

13. Phorbolester oder Zusammensetzung nach Anspruch 10, zur Verwendung nach Anspruch 10, ferner umfassend Verabreichen mindestens eines sekundären oder begleitenden therapeutischen Mittels, das in einer kombinatorischen Formulierung oder einem Regime koordinierter Behandlung mit dem Phorbolester der Formel I wirksam ist, um Auswirkungen eines Schlaganfalls bei dem Individuum zu behandeln oder zu verhüten, wobei das sekundäre oder begleitende therapeutische Mittel gleichzeitig mit, vor, oder nach, Verabreichung des Phorbolesters der Formel I verabreicht wird.

14. Phorbolester oder Zusammensetzung nach Anspruch 13, zur Verwendung nach Anspruch 13, wobei das mindestens eine sekundäre oder begleitende therapeutische Mittel ein Antikoagulans, Statin, Fibrat, Angiotensin-II-Rezeptor-Blocker, ein Thrombozytenaggregationshemmer, Hemmer des Angiotensin konvertierenden Enzyms, Beta-Blocker, Kalziumkanalblocker oder Diuretikum ist.

15. Phorbolester oder Zusammensetzung nach Anspruch 1, zur Verwendung nach Anspruch 1, oder Phorbolester nach Anspruch 10, zur Verwendung nach Anspruch 10, wobei der Phorbolester 12-O-Tetradecanoylphorbol-13-acetat (TPA) ist.

## Revendications

1. Ester de phorbol de la Formule I, ou un sel pharmaceutiquement acceptable ou un énantiomère de celui-ci pour utilisation dans un procédé pour la prévention ou le traitement d'un ou de plusieurs des effets d'un accident vasculaire cérébral chez un sujet mammifère ou composition comprenant un ester de phorbol de la Formule I, ou un sel pharmaceutiquement acceptable ou un énantiomère de celui-ci pour utilisation dans un procédé pour la prévention ou le traitement d'un ou de plusieurs des effets d'un accident vasculaire cérébral chez un sujet mammifère ; 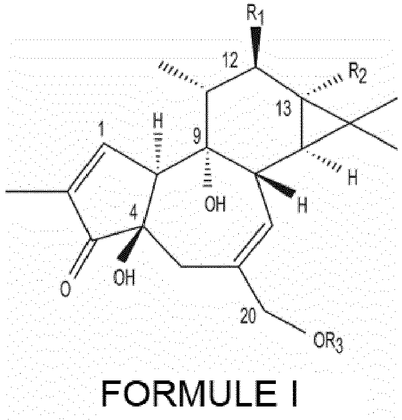
dans lequel R₁ et R₂ sont choisis dans le groupe constitué de : hydrogène, hydroxyle, 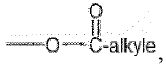 dans lequel le groupe alkyle contient de 1 à 15 atomes de carbone, 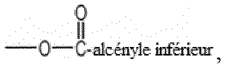 dans lequel le groupe alcényle inférieur contient de 1 à 7 atomes de carbone 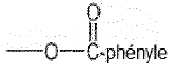 et 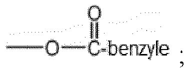 et R₁ est un hydrogène ou 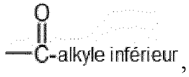 dans lequel le groupe alkyle inférieur contient de 1 à 7 atomes de carbone ;
dans lequel les un ou plusieurs des effets d'un accident vasculaire cérébral sont une paralysie, une défaillance spatiale, un jugement affaibli, un défaut du côté gauche, une perte de mémoire, une aphasie, des problèmes de coordination et d'équilibre, une nausée, un vomissement, un déficit cognitif, une perception affaiblie, une orientation affaiblie, une hémianopsie homonyme ou une impulsivité ; et
dans lequel l'ester de phorbol de la Formule I est dans une quantité de dosage unitaire d'environ 10 µg à environ 1500 µg.

2. Ester de phorbol ou composition selon la revendication 1 pour utilisation selon la revendication 1, dans lequel R₁ ou R₂ est 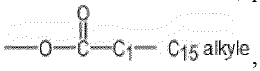 le R₁ ou R₂ restant est 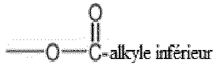 , dans lequel le groupe alkyle inférieur contient de 1 à 7 atomes de carbone, et R₃ est un hydrogène.

3. Ester de phorbol ou composition selon la revendication 1, pour utilisation selon la revendication 1, dans lequel l'ester de phorbol est le 13-butyrate de phorbol, le 12-décanoate de phorbol, le 13-décanoate de phorbol, le 12,13-diacétate de phorbol, le 13,20-diacétate de phorbol, le 12,13-dibenzoate de phorbol, le 12,13-dibutyrate de phorbol, le 12,13-didécanoate de phorbol, le 12,13-dihexanoate de phorbol, le 12,13-dipropionate de phorbol, le 12-myristate de phorbol, le 13-myristate de phorbol, le 12,13,20-triacétate de phorbol, le 13-angélate de 12-désoxyphorbol, le 13-angélate-20-acétate de 12-désoxyphorbol, le 13-isobutyrate de 12-désoxyphorbol, le 13-isobutyrate-20-acétate de 12-désoxyphorbol, le 13-phénylacétate de 12-désoxyphorbol, le 13-phénylacétate-20-acétate de 12-désoxyphorbol, le 13-tétradécanoate de 12-désoxyphorbol, le 12-tigliate-13-décanoate de phorbol, le 13-acétate de 12-désoxyphorbol, le 12-acétate de phorbol ou le 13-acétate de phorbol.

4. Ester de phorbol ou composition selon la revendication 1, pour utilisation selon la revendication 1, dans lequel le procédé ou la composition comprend en outre l'administration d'au moins un agent thérapeutique secondaire ou auxiliaire qui est efficace dans une formulation combinatoire ou un régime de traitement coordonné avec ledit ester de phorbol de la Formule I pour traiter ou prévenir les effets d'un accident vasculaire cérébral chez ledit sujet.

5. Ester de phorbol ou composition selon la revendication 4, pour utilisation selon la revendication 4, dans lequel l'au moins un agent thérapeutique secondaire ou auxiliaire est destiné à être administré audit sujet dans un protocole d'administration coordonné, simultanément avec, avant, ou après, l'administration dudit ester de phorbol audit sujet.

6. Ester de phorbol ou composition selon la revendication 4, pour utilisation selon la revendication 4, dans lequel l'au moins un agent thérapeutique secondaire ou auxiliaire est un activateur tissulaire du plasminogène, un anticoagulant, une statine, un fibrate, des bloqueurs de récepteur de l'angiotensine II, un inhibiteur de l'enzyme de conversion de l'angiotensine, un bêtabloquant, un agent antiplaquettaire, un bloqueur des canaux calciques ou un diurétique.

7. Ester de phorbol ou composition selon la revendication 1, pour utilisation selon la revendication 1, dans lequel le procédé comprend en outre une intervention chirurgicale en combinaison avec l'ester de phorbol de la Formule I pour traiter ou prévenir les effets d'un accident vasculaire cérébral chez ledit sujet.

8. Ester de phorbol ou composition selon la revendication 7, pour utilisation selon la revendication 7, dans lequel l'intervention chirurgicale est une endartériectomie de la carotide, une angioplastie, le placement d'une endoprothèse, une craniotomie, l'insertion d'un pacemaker, l'implantation d'un défibrillateur, le remplacement de valves, un pontage coronarien, une transplantation cardiaque, la mobilisation de rouleau endovasculaire ou la fermeture du foramen ovale perméable.

9. Ester de phorbol ou composition selon l'une quelconque des revendications 1 à 8, pour utilisation selon l'une quelconque des revendications 1 à 8, où le sujet mammifère est un sujet mammifère qui a souffert d'un accident vasculaire cérébral.

10. Ester de phorbol de la Formule I, ou un sel pharmaceutiquement acceptable ou un énantiomère de celui-ci pour utilisation dans un procédé pour la prévention ou le traitement d'un ou de plusieurs symptômes d'un accident vasculaire cérébral chez un sujet mammifère, ou composition comprenant un ester de phorbol de la Formule I, ou un sel pharmaceutiquement acceptable ou un énantiomère de celui-ci pour utilisation dans un procédé pour la prévention ou le traitement d'un ou de plusieurs symptômes d'un accident vasculaire cérébral chez un sujet mammifère ; 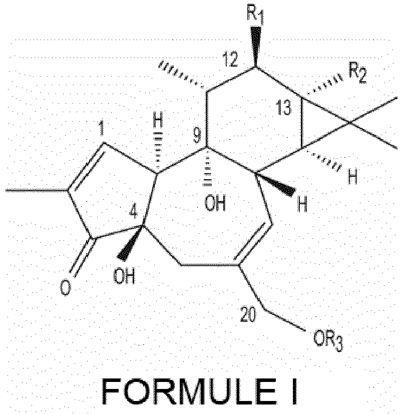
dans lequel R₁ et R₂ sont choisis dans le groupe constitué de : hydrogène, hydroxyle, 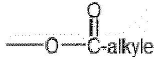 , dans lequel l'alkyle contient de 1 à 15 atomes de carbone, 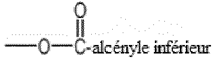 , dans lequel le groupe alcényle inférieur contient de 1 à 7 atomes de carbone, 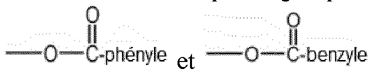 ; et R₃ est un hydrogène et 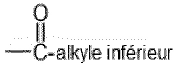 , dans lequel le groupe alkyle inférieur contient de 1 à 7 atomes de carbone ;
dans lequel les un ou plusieurs symptômes d'un accident vasculaire cérébral sont une faiblesse ou un engourdissement soudain sur un côté du corps, une confusion ou un trouble soudain du discours de langage ou de compréhension, un trouble soudain avec une marche, un étourdissement ou une perte d'équilibre ou de coordination ; et
dans lequel l'ester de phorbol de la Formule I est dans une quantité de dosage unitaire d'environ 10 µg à environ 1500 µg.

11. Ester de phorbol ou composition selon la revendication 10, pour utilisation selon la revendication 10, dans lequel R₁ ou R₂ est 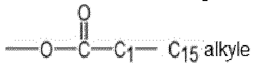 et le R₁ ou R₂ restant est 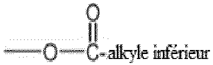 , dans lequel le groupe alkyle inférieur contient de 1 à 7 atomes de carbone, et R₃ est un hydrogène.

12. Ester de phorbol ou composition selon la revendication 10, pour utilisation selon la revendication 10, dans lequel l'ester de phorbol est le 13-butyrate de phorbol, le 12-décanoate de phorbol, le 13-décanoate de phorbol, le 12,13-diacétate de phorbol, le 13,20-diacétate de phorbol, le 12,13-dibenzoate de phorbol, le 12,13-dibutyrate de phorbol, le 12,13-didécanoate de phorbol, le 12,13-dihexanoate de phorbol, le 12,13-dipropionate de phorbol, le 12-myristate de phorbol, le 13-myristate de phorbol, le 12,13,20-triacétate de phorbol, le 13-angélate de 12-désoxyphorbol, le 13-angélate-20-acétate de 12-désoxyphorbol, le 13-isobutyrate de 12-désoxyphorbol, le 13-isobutyrate-20-acétate de 12-désoxyphorbol, le 13-phénylacétate de 12-désoxyphorbol, le 13-phénylacétate-20-acétate de 12-désoxyphorbol, le 13-tétradécanoate de 12-désoxyphorbol, le 12-tigliate-13-décanoate de phorbol, le 13-acétate de 12-désoxyphorbol, le 12-acétate de phorbol ou le 13-acétate de phorbol.

13. Ester de phorbol ou composition selon la revendication 10, pour utilisation selon la revendication 10, comprenant en outre l'administration d'au moins un agent thérapeutique secondaire ou auxiliaire qui est efficace dans une formulation combinatoire ou un régime de traitement coordonné avec ledit ester de phorbol de la Formule I pour traiter ou prévenir les effets d'un accident vasculaire cérébral chez ledit sujet, dans lequel l'agent thérapeutique secondaire ou auxiliaire est administré simultanément avec, avant, ou après, l'administration dudit ester de phorbol de la Formule I.

14. Ester de phorbol ou composition selon la revendication 13, pour utilisation selon la revendication 13, dans lequel l'au moins un agent thérapeutique secondaire ou auxiliaire est un anticoagulant, une statine, un fibrate, des bloqueurs de récepteur de l'angiotensine II, un agent antiplaquettaire, un inhibiteur de l'enzyme de conversion de l'angiotensine, un bêtabloquant, un bloqueur des canaux calciques ou un diurétique.

15. Ester de phorbol ou composition selon la revendication 1, pour utilisation selon la revendication 1 ou ester de phorbol selon la revendication 10, pour utilisation selon la revendication 10, dans lequel l'ester de phorbol est le 12-O-tétradécanoylphorbol-13-acétate (TPA).
